(19) Europäisches Patentamt
European Patent Office
Office européen des brevets

(11) Veröffentlichungsnummer : **0 322 643 B1**

# (12) EUROPÄISCHE PATENTSCHRIFT

(45) Veröffentlichungstag der Patentschrift : **24.07.91 Patentblatt 91/30**

(51) Int. Cl.[5] : **A61K 37/66, // (A61K37/66, 31:445, 31:495)**

(21) Anmeldenummer : **88120884.7**

(22) Anmeldetag : **14.12.88**

## (54) Arzneimittel enthaltend in Kombination Interferon und 1-Desoxy-piperidinosen, Verfahren zu deren Herstellung und deren Verwendung.

Die Akte enthält technische Angaben, die nach dem Eingang der Anmeldung eingereicht wurden und die nicht in dieser Patentschrift enthalten sind.

(30) Priorität : **23.12.87 DE 3743749**

(43) Veröffentlichungstag der Anmeldung : **05.07.89 Patentblatt 89/27**

(45) Bekanntmachung des Hinweises auf die Patenterteilung : **24.07.91 Patentblatt 91/30**

(84) Benannte Vertragsstaaten : **AT BE CH DE ES FR GB GR IT LI NL SE**

(56) Entgegenhaltungen :
**EP-A- 0 000 947**
**CHEMICAL ABSTRACTS, Band 107, Nr. 25, 21. Dezember 1987, Seite 27, Nr. 228527r, Colombus, Ohio, US; P.S. SUNKARA et al.: "Antiretroviral activity of castanospermine and deoxynojirimycin, specific inhibitors of glycoprotein processing", & BIOCHEM. BIOPHYS. RES. COMMUN. 1987, 148(1), 206-10**

(73) Patentinhaber : **BAYER AG**
**W-5090 Leverkusen 1 Bayerwerk (DE)**

(72) Erfinder : **Paessens, Arnold, Dr.**
**Stresemannstrasse 56**
**W-5657 Haan (DE)**
Erfinder : **Schüller, Matthias, Dr. Dr.**
**Gellertweg 20**
**W-5600 Wuppertal 1 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Kombination von N-substituierten 1-Desoxypiperidinosen mit Interferonen und die Verwendung der Kombination in Arzneimitteln, insbesondere in antiviralen Arzneimitteln, mit Wirkung gegen das Virus der humanen Immunschwäche (HIV) und das Krankheitsbild des "Aids Related Complex" (ARC).

Es ist bekannt, daß Derivate von 1-Desoxynojirimycin und 1-Desoxy-mannonojirimycin Glukosidase-Inhibitoren sind und zur Behandlung von Kohlenhydratstoffwechselerkrankungen eingesetzt werden [vgl. EP 947; EP 8058 ; EP 34 784 ; DE 28 48 117 ; EP 193 770]. Darüberhinaus ist bekannt, daß bestimmte 1-Desoxynojirimycin-Derivate herbizide Eigenschaften aufweisen [vgl. DE 30 24 901] und 1-Desoxynojirimycin und 1-Desoxymannonojirimycin und Derivate davon Biosynthese von Glykoproteinen beeinflussen [vgl. U. Fuhrmann, E. Bause, H. Ploegh ; Review ; Biochimica et Biophysica Acta 825, 95-110 (1985)].

Die vorliegende Erfindung betrifft Kombinationen von Interferonen mit N-substituierten Derivaten von 1-Desoxynojirimycin und 1-Desoxy-mannonojirimycin der allgemeinen Formel (I),

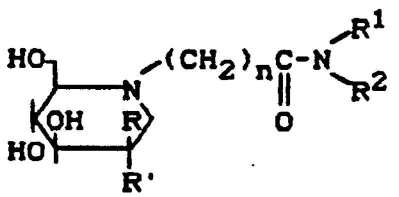

(I)

in welcher der Rest

R oder R′ für eine Hydroxylgruppe steht und der jeweils andere, R′ oder R, für Wasserstoff steht,

n — für eine Zahl ~~2 bis 6~~ steht,

$R^1$ — für Wasserstoff steht, oder
— für geradkettiges oder verzweigtes Alkyl mit bis zu 3 Kohlenstoffatomen oder für Benzyl steht,

und

$R^2$ — für geradkettiges oder verzweigtes Alkyl mit bis zu 8 Kohlenstoffatomen steht, das substituiert sein kann durch Aryl mit 6 bis 10 Kohlenstoffatomen, wobei der Arylrest bis zu 4 gleiche oder verschiedene Substituenten der Reihe Fluor, Chlor, Brom, Iod, Cyano, Nitro, Alkyl, Alkoxy oder Alkythio mit jeweils bis zu 6 Kohlenstoffatomen, Dioxymethylen, Dioxyethylen, Trifluormethyl, Trifluormethoxy, Difluormethoxy, Carboxy oder Alkoxycarbonyl mit bis zu 4 Kohlenstoffatomen tragen kann, oder durch Pyridyl, Thienyl, Furyl, Pyrimidyl, Pyridazinyl, Pyrazinyl oder Chinolyl substituiert sein kann, oder
— für Cycloalkyl mit 3 bis 7 Kohlenstoffatomen steht, oder
— für Aryl mit 6 bis 10 Kohlenstoffatomen steht, wobei der Arylrest bis zu 4-fach gleich oder verschieden substituiert sein kann durch Fluor, Chlor, Brom, Iod, Cyano, Alkyl mit bis zu 6 Kohlenstoffatomen, Alkyloxy mit bis zu 6 Kohlenstoffatomen, Alkylsulfonyl mit jeweils bis zu 4 Kohlenstoffatomen, Trifluormethyl, Trifluormethoxy, Difluormethylen, Difluormethoxy, Trifluormethylthio, Dioxymethylen, Dioxyethylen, durch Cycloalkyl mit 3 bis 7 Kohlenstoffatomen, durch Carboxy oder Alkoxycarbonyl mit bis zu 4 Kohlenstoffatomen, durch Aryloxy mit 6 bis 10 Kohlenstoffatomen, wobei der Arylrest bis zu 4 gleiche oder verschiedene Substituenten der Reihe Fluor, Chlor, Brom, Iod, Cyano, Hydroxy, Nitro, Alkyl, Alkoxy, Alkylthio mit jeweils bis zu 6 Kohlenstoffatomen, Dioxymethylen, Dioxyethylen, Trifluormethyl, Trifluormethoxy oder Difluormethoxy tragen kann, oder durch Halogenalkoxy mit bis zu 6 Kohlenstoffatomen und bis zu 5 Fluor und/oder 3 Chloratomen, oder durch Hydroxyalkoxy, Hydroxyalkyl oder Cyanoalkyl mit jeweils bis zu 8 Kohlenstoffatomen, oder durch Alkenyloxy mit bis zu 6 Kohlenstoffatomen, oder durch eine Gruppe —$XR^3$,

wobei

X — eine geradkettige oder verzweigte Alkylen- oder Alkenylenkette mit bis zu 8 Kohlenstoffatomen bedeutet,

und

$R^3$ — Aryl mit 6 bis 10 Kohlenstoffatomen bedeutet, wobei der Arylrest bis zu 4 gleiche oder verschiedene Substituenten der Reihe Fluor, Chlor, Brom, Iod, Cyano, Alkyl, Alkoxy, Alkylthio mit jeweils bis zu 6 Kohlenstoffatomen, Nitro, Dioxymethylen, Dioxyethylen, Trifluormethyl, Trifluormethoxy, Difluormethoxy oder Trifluormethylthio tragen kann, oder
— einen 5- bis 6-gliedrigen gesättigten oder ungesättigten heterocyclischen Ring bedeutet, der benzokondensiert sein kann und der als Heteroatome Sauerstoff, Schwefel oder bis zu 2 Stickstoffatome enthalten kann, oder
— Hydroxy, Carboxy, Alkoxycarbonyl mit bis zu 6 Kohlenstoffatomen, Alkylcarbonyloxy mit bis zu 18 Kohlenstoffatomen, Cycloalkyl mit 3 bis 6 Kohlenstoffatomen bedeutet, oder
— eine Gruppe $—OSO_3H$, $—CONH\text{-}NH_2$, $—CONH_2$ oder Amino, Alkylamino oder Dialkylamino mit jeweils bis zu 6 Kohlenstoffatomen je Alkylgruppe bedeutet, oder
— Alkyl oder Hydroxyalkyl mit bis zu 8 Kohlenstoffatomen bedeutet,

oder

$R^1$ und $R^2$ gemeinsam mit dem Stickstoffatom einen 5- bis 6-gliedrigen Ring bilden, der durch Sauerstoff oder die Gruppe $—NR^4$ unterbrochen sein kann, und der substituiert sein kann durch Alkyl mit bis zu 4 Kohlenstoffatomen, Hydroxy, Phenyl, Carboxy oder Alkoxycarbonyl mit bis zu 4 Kohlenstoffatomen,

wobei

$R^4$ — Aryl mit 6 bis 10 Kohlenstoffatomen bedeutet, wobei der Arylrest bis 4 gleiche oder verschiedene Substituenten der Gruppe Fluor, Chlor, Brom, Iod, Cyano, Nitro, Hydroxy, Alkyl, Alkoxy, Alkylthio mit jeweils bis zu 6 Kohlenstoffatomen, Dioxymethylen, Dioxyethylen, Trifluormethyl, Trifluormethoxy oder Difluormethoxy tragen kann, oder
— Alkoxycarbonyl mit bis zu 6 Kohlenstoffatomen bedeutet, oder
— Pyridyl, Pyrimidyl, Furyl, Thienyl, Pyrazinyl, Pyridazinyl oder Chinolyl bedeutet, oder
— Cycloalkyl mit 3 bis 7 Kohlenstoffatomen bedeutet, oder
— Alkyl oder Alkenyl mit bis zu 6 Kohlenstoffatomen bedeutet, das bis zu 2-fach gleich oder verschieden substituiert sein kann durch gegebenenfalls durch Chlor, Dioxymethylen oder Trifluormethyl substituiertes Phenyl, Hydroxy, Amino, Alkylamino, Dialkylamino mit jeweils bis zu 3 Kohlenstoffatomen je Alkylgruppe, Fluor, Chlor, Brom, Cycloalkyl mit 3 bis 6 Kohlenstoffatomen, durch Carboxy oder Alkoxycarbonyl mit bis zu 6 Kohlenstoffatomen, durch Pyridyl, Pyrimidyl, Pyrazinyl, Morpholinyl, Thiomorpholinyl oder Pyrrolidinyl, durch Morpholinocarbonyl, durch Alkylaminocarbonyl, Dialkylaminocarbonyl oder Phenylalkylaminocarbonyl mit jeweils bis zu 6 Kohlenstoffatomen je Alkylgruppe.

Bevorzugt seien Verbindungen der allgemeinen Formel (I) genannt, in welcher

n — für die Zahl 2 bis 4 steht,
$R^1$ — für Wasserstoff steht,

und

$R^2$ — für geradkettiges oder verzweigtes Alkyl mit bis zu 6 Kohlenstoffatomen steht, das substituiert sein kann durch Pyridyl, oder durch Phenyl, wobei der Phenylrest bis zu 3 gleiche oder verschiedene Substituenten der Reihe Fluor, Chlor, Brom, Hydroxy, Alkyl, Alkoxy mit bis zu 4 Kohlenstoffatomen, Dioxymethylen, Dioxyethylen oder Carboxy tragen kann, oder
— für Cyclopentyl, Cyclohexyl oder Cycloheptyl steht, oder
— für Phenyl steht, das bis zu 3-fach gleich oder verschieden substituiert sein kann durch Fluor, Chlor, Brom, Cyano, Alkyl mit bis zu 4 Kohlenstoffatomen, Hydroxyalkyl oder Hydroxyalkoxy mit bis zu 4 Kohlenstoffatomen, Trifluormethyl, Trifluormethoxy, Dioxymethylen, Dioxyethylen, durch gegebenenfalls im Aromaten durch Fluor, Chlor, Brom, Hydroxy, Alkyl, Alkoxy mit bis zu 4 Kohlenstoffatomen, Dioxymethylen, Trifluormethyl oder Carboxy substituiertes Phenoxy, oder durch geradkettiges oder verzweigtes Alkenyloxy mit bis zu 4 Kohlenstoffatomen, oder durch eine Gruppe $-XR^3$

wobei

X — eine geradkettige oder verzweigte Alkylenkette mit bis zu 6 Kohlenstoffatomen bedeutet

und

$R^3$ — ein bis zu 3-fach gleich oder verschieden durch Fluor, Chlor, Brom, Hydroxy, Alkyl, Alkoxy mit bis zu 4 Kohlenstoffatomen, Dioxymethylen, Trifluormethyl oder Carboxy substituiertem Phenyl bedeutet, oder
— Pyridyl, Morpholinyl oder Piperidinyl, bedeutet, oder
– Hydroxy, Carboxy, Alkoxycarbonyl mit bis zu 4 Kohlenstoffatomen, oder Cyclopropyl, Cyclopentyl oder Cyclohexyl bedeutet, oder
– Amino, Alkylamino oder Dialkylamino mit jeweils bis zu 4 Kohlenstoffatomen je Alkylgruppe bedeutet,

oder

$R^1$ und $R^2$ gemeinsam mit dem Stickstoff einen Ring der Formel

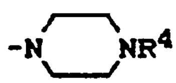 , 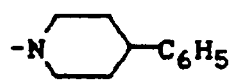 , 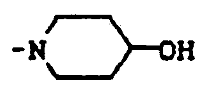 ,

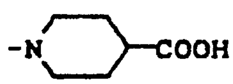 oder 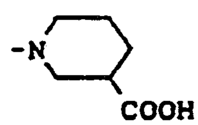

bilden, wobei

$R^4$ — einen gegebenenfalls bis zu 3-fach gleich oder verschieden durch Fluor, Chlor, Brom, Hydroxy, Alkyl, Alkoxy mit bis zu 4 Kohlenstoffatomen, Dioxymethylen, Trifluormethyl oder Carboxy substituertes Phenyl bedeutet, oder
— Alkoxycarbonyl mit bis zu 4 Kohlenstoffatomen bedeutet, oder
— Pyridyl oder Pyrimidyl bedeutet, oder
— Cyclopropyl, Cyclopentyl oder Cyclohexyl bedeutet, oder
— geradkettiges oder verzweigtes Alkyl oder Alkenyl mit bis zu 4 Kohlenstoffatomen bedeutet, das bis zu 2-fach gleich oder verschieden durch gegebenenfalls durch Chlor, Trifluormethyl oder Dioxymethylen substituiertes Phenyl, Hydroxy, Amino, Methylamino, Dimethylamino, Fluor, Chlor, Cyclopropyl, Cyclopentyl, Cyclohexyl oder Alkoxycarbonyl mit bis zu 4 Kohlenstoffatomen, durch Morpholinyl, Pyrrolidinyl, Morpholinocarbonyl, Dialkylamino oder Phenylalkylamino mit jeweils bis zu 4 Kohlenstoffatomen substituiert sein kann.

Besonders bevorzugt seien Verbindungen der allgemeinen Formel (I) genannt, in welcher

n — für die Zahl 2 steht,
$R^1$ — für Wasserstoff steht,

und

$R^2$ — für Phenyl steht, das bis zu 3-fach gleich oder verschieden substituiert sein kann durch Fluor, Chlor, Brom, Cyano, Alkyl mit bis zu 4 Kohlenstoffatomen, Hydroxyalkyl, Hydroxyalkoxy mit bis zu 4 Kohlenstoffatomen, Alkylthio mit bis zu 4 Kohlenstoffatomen, Trifluormethyl, Trifluormethoxy,

oder

$R^1$ und $R^2$ gemeinsam mit dem Stickstoff einen Ring der Formel

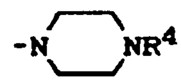 bilden,

wobei

$R^4$ — einen gegebenenfalls bis zu 2-fach gleich oder verschieden durch Fluor, Chlor, Brom, Hydroxy, Alkyl, Alkoxy mit bis zu 4 Kohlenstoffatomen, substituiertes Phenyl bedeutet, oder
— Alkoxycarbonyl mit bis zu 4 Kohlenstoffatomen bedeutet, oder
— Cyclopropyl, Cyclopentyl oder Cyclohexyl bedeutet, oder
— geradkettiges oder verzweigtes Alkyl oder Alkenyl mit bis zu 4 Kohlenstoffatomen bedeutet, das bis zu 2-fach gleich oder verschieden durch gegebenenfalls durch Chlor, Trifluormethyl oder Dioxymethylen, substituiertes Phenyl, Hydroxy, Amino, Dimethylamino, Cyclopropyl oder Alkoxycarbonyl mit bis zu 4 Kohlenstoffatomen, durch Morpholinyl oder Pyrrolidinyl substituiert sein kann.

Ganz besonders bevorzugt seien folgende Verbindungen genannt:
3-(1,5-Didesoxy-1,5-imino-D-glucit-N-yl)-propionsäure(4-methoxy)-anilid,
3-(1,5-Didesoxy-1,5-imino-D-glucit-N-yl)-propionsäure(4-methoxycarbonyl)-anilid,
3-(1,5-Didesoxy-1,5-imino-D-glucit-N-yl)-propionsäure-4-(4-pyridylmethoxy)-anilid,
3-(1,5-Didesoxy-1,5-imino-D-glucit-N-yl)-propionsäure-4-benzyloxy-anilid,
3-(1,5-Didesoxy-1,5-imino-D-glucit-N-yl)-propionsäure-4-trifluormethoxy-anilid,
3-(1,5-Didesoxy-1,5-imino-D-glucit-N-yl)-propionsäure-4-(2-oxocyclohexyloxy)-anilid,
3-(1,5-Didesoxy-1,5-imino-D-glucit-N-yl)-propionsäure-4-tert.butyl-anilid,
3-(1,5-Didesoxy-1,5-imino-D-glucit-N-yl)-propionsäure-4-(6-methyl-heptyloxy)-anilid,
3-(1,5-Didesoxy-1,5-imino-D-glucit-N-yl)-propionsäure-4-(2-N,N-diethyl-amino-ethoxy)-anilid,
3-(1,5-Didesoxy-1,5-imino-D-glucit-N-yl)-propionsäure-4-allyloxy-anilid,
3-(1,5-Didesoxy-1,5-imino-D-glucit-N-yl)-propionsäure-4-trifluormethyl-anilid,
3-(1,5-Didesoxy-1,5-imino-D-glucit-N-yl)-propionsäure-4-(2-chlor-1,1,2-trifluorethoxy)-anilid,
3-(1,5-Didesoxy-1,5-imino-D-glucit-N-yl)-propionsäure-4-isopropyl-anilid,
3-(1,5-Didesoxy-1,5-imino-D-glucit-N-yl)-propionsäure-2-isopropyl-anilid,
3-(1,5-Didesoxy-1,5-imino-D-glucit-N-yl)-propionsäure-4-fluor-anilid,
3-(1,5-Didesoxy-1,5-imino-D-glucit-N-yl)-propionsäure-2-fluor-anilid,
1-[3-(1,5-Didesoxy-1,5-imino-D-glucit-N-yl)-propionyl]-2,3-dihydroindol,
3-(1,5-Didesoxy-1,5-imino-D-glucit-N-yl)-propionsäure-2,4-difluoranilid,
3-(1,5-Didesoxy-1,5-imino-D-glucit-N-yl)-propionsäure-2-trifluormethyl-anilid,
3-(1,5-Didesoxy-1,5-imino-D-glucit-N-yl)-propionsäure-2,6-difluoranilid,
3-(1,5-Didesoxy-1,5-amino-D-glucit-N-yl)-propionsäure-(adamantan-1-yl)-amid,
3-(1,5-Didesoxy-1,5-imino-D-glucit-N-yl)-propionsäure-3,4,5-trimethoxy-anilid,
3-(1,5-Didesoxy-1,5-imino-D-glucit-N-yl)-propionsäure-3,4-dimethoxy-anilid,
3-(1,5-Didesoxy-1,5-imino-D-glucit-N-yl)-propionsäure-3,5-dimethoxy-anilid,
3-(1,5-Didesoxy-1,5-imino-D-glucit-N-yl)-propionsäure-2,4,6-tribromanilid,
3-(1,5-Didesoxy-1,5-imino-D-glucit-N-yl)-propionsäure-3-cyano-anilid,
3-(1,5-Didesoxy-1,5-imino-D-glucit-N-yl)-propionsäure-4-cyclohexyl-anilid,
3-(1,5-Didesoxy-1,5-imino-D-glucit-N-yl)-propionsäure-3,4-ethylendioxy-anilid,
3-(1,5-Didesoxy-1,5-imino-D-glucit-N-yl)-propionsäure-2-bromanilid,
3-(1,5-Didesoxy-1,5-imino-D-glucit-N-yl)-propionsäure-3,4-methylendioxy-anilid,
3-(1,5-Didesoxy-1,5-imino-D-glucit-N-yl)-propionsäure-2-methylthio-anilid,
3-(1,5-Didesoxy-1,5-imino-D-glucit-N-yl)-propionyl-4-(4-trifluormethyl-phenyl)-piperazin,
3-(1,5-Didesoxy-1,5-imino-D-glucit-N-yl)-propionsäure-anilid,
3-(1,5-Didesoxy-1,5-imino-D-glucit-N-yl)-propionsäure-4-ethoxy-anilid,
3-(1,5-Didesoxy-1,5-imino-D-glucit-N-yl)-propionsäure-2-dimethylaminoethyl-amid,
3-(1,5-Didesoxy-1,5-imino-D-glucit-N-yl)-propionsäure-[2-(3-methoxyphenyl)ethyl)-amid,
3-(1,5-Didesoxy-1,5-imino-D-glucit-N-yl)-propionsäure-2-pyridylmethyl-amid,
3-(1,5-Didesoxy-1,5-imino-D-glucit-N-yl)-propionyl-(4-cinnamyl)-piperazin,
3-(1,5-Didesoxy-1,5-imino-D-glucit-N-yl)-propionsäure-4-(2-hydroxyethoxy)-anilid,
3-(1,5-Didesoxy-1,5-imino-D-mannit-N-yl)-propionsäure-(4-methoxy)-anilid,

3-(1,5-Didesoxy-1,5-imino-D-mannit-N-yl)-propionsäure-(4-methoxycarbonyl)-anilid,
3-(1,5-Didesoxy-1,5-imino-D-mannit-N-yl)-propionsäure-4-(4-pyridylmethoxy)-anilid,
3-(1,5-Didesoxy-1,5-imino-D-mannit-N-yl)-propionsäure-4-benzyloxy-anilid,
3-(1,5-Didesoxy-1,5-imino-D-mannit-N-yl)-propionsäure-4-trifluormethoxy-anilid,
3-(1,5-Didesoxy-1,5-imino-D-mannit-N-yl)-propionsäure-4-(2-oxocyclohexyloxy)-anilid,
3-(1,5-Didesoxy-1,5-imino-D-mannit-N-yl)-propionsäure-4-tert.butyl-anilid,
3-(1,5-Didesoxy-1,5-imino-D-mannit-N-yl)-propionsäure-4-(6-methyl-heptyloxy)-anilid,
3-(1,5-Didesoxy-1,5-imino-D-mannit-N-yl)-propionsäure-4-(2-N,N-diethyl-amino-ethoxy)-anilid,
3-(1,5-Didesoxy-1,5-imino-D-mannit-N-yl)-propionsäure-4-allyloxy-anilid,
3-(1,5-Didesoxy-1,5-imino-D-mannit-N-yl)-propionsäure-4-trifluormethyl-anilid,
3-(1,5-Didesoxy-1,5-imino-D-mannit-N-yl)-propionsäure-4-(2-chlor-1,1,2-trifluorethoxy)-anilid,
3-(1,5-Didesoxy-1,5-imino-D-mannit-N-yl)-propionsäure-4-isopropyl-anilid,
3-(1,5-Didesoxy-1,5-imino-D-mannit-N-yl)-propionsäure-2-isopropyl-anilid,
3-(1,5-Didesoxy-1,5-imino-D-mannit-N-yl)-propionsäure-4-fluor-anilid,
3-(1,5-Didesoxy-1,5-imino-D-mannit-N-yl)-propionsäure-2-fluor-anilid,
1-[3-(1,5-Didesoxy-1,5-imino-D-mannit-N-yl)-propionyl]-2,3-dihydroindol,
3-(1,5-Didesoxy-1,5-imino-D-mannit-N-yl)-propionsäure-2,4-difluoranilid,
3-(1,5-Didesoxy-1,5-imino-D-mannit-N-yl)-propionsäure-2-trifluormethyl-anilid,
3-(1,5-Didesoxy-1,5-imino-D-mannit-N-yl)-propionsäure-2,6-difluoranilid,
3-(1,5-Didesoxy-1,5-imino-D-mannit-N-yl)-propionsäure-(adamantan-1-yl)-amid,
3-(1,5-Didesoxy-1,5-imino-D-mannit-N-yl)-propionsäure-3,4,5-trimethoxy-anilid,
3-(1,5-Didesoxy-1,5-imino-D-mannit-N-yl)-propionsäure-3,4-dimethoxy-anilid,
3-(1,5-Didesoxy-1,5-imino-D-mannit-N-yl)-propionsäure-3,5-dimethoxy-anilid,
3-(1,5-Didesoxy-1,5-imino-D-mannit-N-yl)-propionsäure-2,4,6-tribromanilid,
3-(1,5-Didesoxy-1,5-imino-D-mannit-N-yl)-propionsäure-3-cyano-anilid,
3-(1,5-Didesoxy-1,5-imino-D-mannit-N-yl)-propionsäure-4-cyclohexyl-anilid,
3-(1,5-Didesoxy-1,5-imino-D-mannit-N-yl)-propionsäure-3,4-ethylendioxy-anilid,
3-(1,5-Didesoxy-1,5-imino-D-mannit-N-yl)-propionsäure-2-bromanilid,
3-(1,5-Didesoxy-1,5-imino-D-mannit-N-yl)-propionsäure-3,4-methylendioxy-anilid,
3-(1,5-Didesoxy-1,5-imino-D-mannit-N-yl)-propionsäure-2-methylthio-anilid,
3-(1,5-Didesoxy-1,5-imino-D-mannit-N-yl)-propionyl-4-(4-trifluormethyl-phenyl)-piperazin,
3-(1,5-Didesoxy-1,5-imino-D-mannit-N-yl)-propionsäure-anilid,
3-(1,5-Didesoxy-1,5-imino-D-mannit-N-yl)-propionsäure-4-ethoxy-anilid,
3-(1,5-Didesoxy-1,5-imino-D-mannit-N-yl)-propionsäure-2-dimethylaminoethyl-amid,
3-(1,5-Didesoxy-1,5-imino-D-mannit-N-yl)-propionsäure-[2-(3-methoxyphenyl)ethyl)-amid,
3-(1,5-Didesoxy-1,5-imino-D-mannit-N-yl)-propionsäure-2-pyridylmethyl-amid,
3-(1,5-Didesoxy-1,5-imino-D-mannit-N-yl)-propionyl-(4-cinnamyl)-piperazin und
3-(1,5-Didesoxy-1,5-imino-D-mannit-N-yl)-propionsäure-4-(2-hydroxyethoxy)-anilid.

Im Rahmen von Untersuchungen, die zu der vorliegenden Erfindung führten, wurde überraschenderweise gefunden, daß Kombinationen der oben genannten Verbindungen der allgemeinen Formel (I) mit Interferonen eine außerordentlich gute Wirkung gegen Retro-Viren besitzen.

Die Verbindungen gemäß Formel (I) können hergestellt werden, indem man
Carbonsäuren der allgemeinen Formel (II)

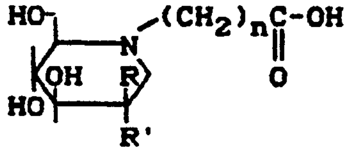

(II)

in welcher

n die oben angegebene Bedeutung hat,

nach bekannten Methoden, gegebenenfalls über ein reaktives Säurederivat, mit Aminen der allgemeinen Formel (III)

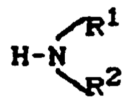 (III),

in welcher

$R^1$ und $R^2$ die oben angegebene Bedeutung haben,
gegebenenfalls in Anwesenheit eines inerten organischen Lösemittels umsetzt.

Als reaktive Säurederivate seien beispielsweise genannt : aktivierte Ester, Hydroxysuccinimidester, Säureimidazolide, Säurehalogenide, gemischte Anhydride, oder die Umsetzung in Anwesenheit von Carbodiimiden wie beispielsweise Cyclohexylcarbodiimid.

Dieses Herstellungsverfahren kann beispielsweise durch folgendes Formelschema erläutert werden :

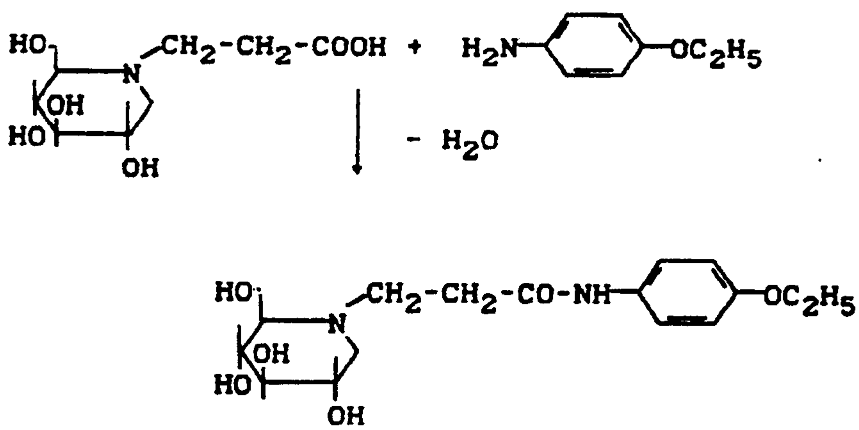


Die Durchführung des Verfahrens lehnt sich an die literaturbekannte Methodik zur Überführung von Carbonsäuren in Carbonsäureamide an. Dabei wird die Carbonsäure zunächst in eine aktivierte Form wie z.B. das Säurechlorid oder das Imidazolid überführt, die entweder als solche isoliert und in einem zweiten Reaktionsschritt umgesetzt werden, oder die in situ direkt zu den erfindungsgemäßen Verbindungen amidiert werden. Als aktivierende Reagenzien seien neben den anorganischen Halogeniden wie Thionylchlorid, Phosphortrichlorid oder Phosphorpentachlorid, oder dem Carbonyldiimidazol, Carbodiimide wie Dicyclohexylcarbodiimid oder 1-Cyclohexyl-3-[2-(N-methyl-morpholino)ethyl] carbodiimid-p-toluolsulfonat oder N-Hydroxyphthalimid oder N-Hydroxy-benztriazol in Gegenwart von Dicyclohexylcarbodiimid beispielhaft erwähnt. Naturgemäß lassen sich die Piperidin-N-yl-carbonsäuren auch in Form ihrer Salze einsetzen. [Die Methodik der Amidierung wird beispielsweise beschrieben : Fieser & Fieser, Reagents for Organic Synthesis, John Wiley & Sons Inc. (1967), Seite 231-236 ; J.C. Shihan and G.P. Hess, J. Am. Chem. Soc. 77, 1067 (1955) ; U. Goodman, G.W. Kenner, Adv. in Protein Chem. 12, 488 (1957) ; W.A. Bonner, P.I. McNamee, J. Org. Chem. 26, 254 (1961) ; H.A. Staab, Angew. Chemie Int. Ed. 1, 351 (1962) ; Fieser & Fieser, Reagents for Organic Synthesis, John Wiley & Sons Inc. 1967, 116, 114 ; H.C. Beyerman, U.O. van der Brink, Re. Trav. 80, 1372 (1961) ; C.A. Buehler, D.E. Pearson, John Wiley & Sons, Volume I (1970), Seite 895 ff, Volume II, (1977)].

Als Lösemittel für das Verfahren kommen neben Wasser alle inerten organischen Lösemittel in Frage, die sich unter den Reaktionsbedingungen nicht verändern. Hierzu gehören vorzugsweise Ether wie Diethylether, Dioxan, Tetrahydrofuran, Glykolmonomethylether oder Glykoldimethylether, oder halogenierte Kohlenwasserstoffe wie Dichlormethan, Trichlormethan oder Tetrachlormethan, oder Amide wie Dimethylformamid, Dimethylacetamid oder Hexamethylphosphorsäuretriamid, oder Kohlenwasserstoffe wie Benzol, Toluol oder Xylol, oder Acetonitril, Nitro methan, Pyridin, Dimethylsulfoxid oder Essigester. Ebenso können Gemische der genannten Lösemittel verwendet werden.

Die Reaktionstemperaturen können in einem breiten Bereich variiert werden. Im allgemeinen arbeitet man in einem Bereich von −70°C bis +140°C, bevorzugt von −20°C bis +100°C.

Die Umsetzung kann bei Normaldruck, aber auch bei erhöhtem oder erniedrigtem Druck durchgeführt werden. Im allgemeinen arbeitet man bei Normaldruck.

Bei der Druchführung des Verfahrens ist das Verhältnis der an der Reaktion beteiligten Stoffe beliebig. Im allgemeinen arbeitet man mit molaren Mengen der Reaktanden. Es hat sich jedoch als günstig erwiesen, das Amin in einem 5-bis 10-fach molaren Überschuß einzusetzen. Besonders zweckmäßig wird das Amin in einem großen Überschuß direkt als Lösemittel eingesetzt.

Die als Ausgangsstoffe eingesetzten D-gluco konfigurierten Carbonsäuren von 1-Desoxy-nojirimycin sind bekannt [DE 30 24 901 ; DE 27 58 025 ; DE 29 22760 ; JP 81/103 153].

Die als Ausgangsstoffe eingesetzten D-manno konfigurierten Carbonsäuren von 1-Desoxy-nojirimycin und ihre Herstellung sind neu.

Als Carbonsäuren werden beispielsweise erfindungsgemäß verwendet :

N-[2-Carboxyethyl]-1-desoxy-nojirimycin,
N-[3-Carboxypropyl]-1-desoxy-nojirimycin,
N-[4-Carboxybutyl]-1-desoxy-nojirimycin,
N-[2-Carboxyethyl]-1-desoxy-manno-nojirimycin,
N-[3-Carboxypropyl]-1-desoxy-manno-nojirimycin oder
N-[4-Carboxybutyl]-1-desoxy-manno-nojirimycin.

Die als Ausgangsstoffe eingesetzten Amine sind bekannt oder können nach bekannten Methoden hergestellt werden wie sie z.B. in Beilstein's Handbuch der Organischen Chemie beschrieben werden.

Als Amine werden beispielsweise erfindungsgemäß verwendet :

2-Fluoranilin,
4-Fluoranilin,
2-Bromanilin,
4-Iodanilin,
2-Trifluormethyl-anilin,
3-Trifluormethyl-anilin,
4-Trifluormethyl-anilin,
3-Cyan-anilin,
2-Methylmercapto-anilin,
3,4-Methylendioxy-anilin,
3,4-Ethylendioxy-anilin,
3,4-Dimethoxy-anilin,
3,5-Dimethoxy-anilin,
3,4,5-Trimethoxy-anilin,
2,6-Difluoranilin,
2,4-Difluoranilin,
3,4-Difluoranilin,
2,4,6-Tribromanilin,
2-Isopropyl-anilin,
4-Isopropyl-anilin,
4-(t-Butyl)-anilin,
4-Cyclohexyl-anilin,
2,3-Dihydroindol,
1,2,3,4-Tetrahydroisochinolin,
Thiomorpholin,
2,2-Dimethyl-1-propylamin,
2-Ethyl-butylamin,
Cyclopentylamin,
Cycloheptylamin,
1-Phenyl-piperazin,
1-(2-Methylphenyl)-piperazin,
1-(3-Methylphenyl)-piperazin,
1-(4-Methylphenyl)-piperazin,
1-(2-Ethylphenyl)-piperazin,
1-(2-Chlorphenyl)-piperazin,
1-(3-Chlorphenyl)-piperazin,
1-(2-Fluorphenyl)-piperazin,
1-(4-Fluorphenyl)-piperazin,
1-(2-Cyanphenyl)-piperazin,
1-(2-Nitrophenyl)-piperazin,

1-(4-Nitrophenyl)-piperazin,
1-(-2-Methoxyphenyl)-piperazin,
1-(3-Methoxyphenyl)-piperazin,
1-(4-Methoxyphenyl)-piperazin,
1-(2-Ethoxyphenyl)-piperazin,
1-(2-Methylmercaptophenyl)-piperazin,
1-(2-Hydroxyphenyl)-piperazin,
1-(4-Hydroxyphenyl)-piperazin,
1-(4-Trifluormethylphenyl)-piperazin,
1-(3-Trifluormethylphenyl)-piperazin,
1-(3-Trifluormethyl-4-chlorphenyl)-piperazin,
1-(2,3-Dimethylphenyl)-piperazin,
1-(2,6-Dimethylphenyl)-piperazin,
1-(3,4-Dimethylphenyl)-piperazin,
1-(3,5-Dichlorphenyl)-piperazin,
1-(3,4-Methylendioxyphenyl)-piperazin,
1-(2,4-Dimethoxyphenyl)-piperazin,
1-(3,4-Dimethoxyphenyl)-piperazin,
1-(3,5-Dimethoxyphenyl)-piperazin,
1-(3,4,5-Trimethoxyphenyl)-piperazin,
1-(2-Pyridyl)-piperazin,
1-(4-Pyridyl)-piperazin,
1-(2-Pyrimidyl)-piperazin,
1-(2-Pyrazinyl)-piperazin,
1-Benzyl-piperazin,
Piperazin-N-carbonsäureethylester,
1-(3,4-Methylendioxybenzyl)-piperazin,
1-(4-Chlor-benzyl)-piperazin,
1-(2-Phenylethyl)-piperazin,
1-(1-Phenylethyl)-piperazin,
1-Benzhydrylpiperazin,
1-(4,4'-Difluor-benzhydryl)-piperazin,
1-Cinnamyl-piperazin,
1-Allyl-piperazin,
1-Isopropyl-piperazin,
1-Cyclopropyl-piperazin,
1-Cyclobutyl-piperazin,
1-Cyclopentyl-piperazin,
1-Cyclohexyl-piperazin,
1-Cycloheptyl-piperazin,
1-(2-Cyclohexyl-ethyl)-piperazin,
1-(2-Dimethylamino-ethyl)-piperazin,
1-(2-Diethylamino-ethyl)-piperazin,
1-(3-Hydroxypropyl)-piperazin,
1-(3-Chorpropyl)-piperazin,
Piperazino-essigsäuremorpholid,
Piperazino-essigsäurepyrrolidid,
Piperazino-essigsäure-N-methyl-anilid,
Piperazino-essigsäureethylester,
4-Phenyl-piperidin,
4-Hydroxy-piperidin,
Piperidin-4-carbonsäure,
Piperidin-3-carbonsäure,
4-Fluorbenzylamin,
2-Chlorbenzylamin,
3-Chlorbenzylamin,
4-Brombenzylamin,
2-Methyl-benzylamin,

4-(t-Butyl)-benzylamin,
2-Methoxy-benzylamin,
3-Methoxy-benzylamin,
4-Methoxy-benzylamin,
3,4-Methylendioxy-benzylamin,
3,4-Dimethoxy-benzylamin,
3,4,5-Trimethoxy-benzylamin,
4-Trifluormethyl-benzylamin,
4-Carboxy-benzylamin,
3,5-Dimethyl-benzylamin,
D(+)-1-Phenylethylamin,
L(−)-1-Phenyl-ethylamin,
2-Aminomethyl-pyridin,
2-Aminomethyl-thiophen,
2-Cyclohexylsulfonylanilin,
2-(3,4-Dichlorbenzylsulfonyl)-anilin,
2-(3,4-Dichlorbenzylsulfonyl)-5-acetylamino-anilin,
2-(3,4-Dichlorbenzylsulfonyl)-4-acetylamino-anilin,
2-(Phenylsulfonyl)-4-acetylamino-anilin,
2-(3,4-Dichlorbenzylsulfonyl)-4-methyl-anilin,
4-(1-Methyl-2-hydroxy-ethoxy)-anilin,
4-(Cyclohexylmethoxy)-anilin,
(5-Ethylsulfonyl-1,3,4-thiadiazol-2-yl)-amin,
(5,6-Dihydro-4H-1,3-oxazin-2-yl)-amin,
2-Amino-4-trifluormethyl-pyrimidin,
2-Hydrazino-4-trifluormethyl-pyrimidin,
4,5-Bistrifluormethyl-2-hydrazino-pyrimidin,
4,6-Bistrifluormethyl-2-hydrazino-pyrimidin,
3,4-Bistrifluormethyl-anilin,
3,5-Bistrifluormethyl-anilin,
2-(2-Methoxycarbonyl-phenylthio)-5-methyl-anilin,
2,6-Dichlor-4-isobutoxycarbonyl-anilin,
4-Isobutoxycarbonyl-anilin,
2-Amino-5-dimethylamino-1,3,4-thiadiazol,
1-Amino-4-hydroxymethyl-piperidin,
6-Amino-5-chlor-8-methoxy-2-methyl-chinolin,
4-(5-Methylhexyloxy)-anilin oder
4-(Pyridylmethoxy)-anilin.

Für die obengenannten Verbindungen allein wurde bereits eine gute antiretrovirale Wirkung gefunden, wie sie in DE 37 36 771 beschrieben wird.

Überraschenderweise wurde jetzt entdeckt, daß Kombinationen von N-substituierten 1-Desoxypiperidinosen der allgemeinen Formel (I) mit Interferonen eine synergistische antivirale Wirkung insbesondere gegen Retroviren aufweisen, die weit über die Summe der Wirkungen der einzelnen antiviralen Wirkstoffe allein hinausgeht.

Ein besonderer Vorteil der erfindungsgemäßen Kombinationen ist, daß sie es ermöglichen, bei geringer Dosierung der Einzelkomponenten eine gleiche oder bessere antivirale Wirkung zu erzielen, wodurch der therapeutische Index vergrößert wird : So erlauben die erfindungsgemäßen Kombinationen den Einsatz der antiviralen Wirkstoffe zur Behandlung von Krankheitszuständen wie AIDS, ARC oder anderen retrovirusinduzierten Erkrankungen des lymphatischen Systems und/oder anderer Systeme des menschlichen oder tierischen Organismus, bei denen der Einsatz der Einzelkomponenten allein eine wesentlich höhere Dosierung des Wirkstoffs erforderlich machen würde, was zu unerwünschten Nebenwirkungen führen könnte.

Die Verwendung der erfindungsgemäßen Kombinationen bedeutet deshalb einen erheblichen therapeutischen Vorteil bei der Behandlung der obengenannten Krankheitsbilder.

Dies wird beispielhaft durch die weiter unten angegebenen Versuchsdaten für die Wirkungen der Kombinationen von Verbindungen der allgemeinen Formel (I) und Interferon am Visna-Virus in Zellkulturen belegt. Das Visna-Virus und das HIV Virus (Virus der humanen Immundefiziens) gehören beide zu der Retro-Virus-Subfamilie der Lentiviren [vgl. Haase, A.T., Nature (1986) 332, 130-136]. Beide Viren weise eine ähnliche Genomorganisation und ein gegenüber den anderen Retroviren komplexes Transkriptionsmuster auf [vgl.

Sonijo, P. et al., Cell (1985), 42, (1987) 61 (5) 1325-1331].

In Zellkulturen, die mit Visna-Virus infiziert sind, treten 5 bis 10 Tage nach der Infektion ausgeprägte virusinduzierte, zytopathische Effekte auf. Durch Behandlung der infizierten Zellkulturen mit den erfindungsgemäßen Kombinationen von Verbindungen der allgemeinen Formel (I) und Interferon konnte das Auftreten dieser zytopathischen Effekte verhindert werden.

Der Visna-Virus-Test wurde nach der Methode von 0. Narayan et al., Journal of Infectious Diseases 135, 5, 1977, 800-806 durchgeführt. Dazu wurden die erfindungsgemäßen Kombinationen von Verbindungen der allgemeinen Formel (I) und Interferon im Kulturmedium zu nicht zytotoxischen Konzentrationen verdünnt, und zwar in 96er Microtiterplatten. Anschließend wurden zu jeder Verdünnung der Wirkstoffe Schafsfibroblastenzellen ($5 \times 10^4$ Zellen pro Napf) in Kulturmedium zugeführt.

Jedes Näpfchen erhielt dann 50 µl einer Visna-Viruslösung mit einem Titer von ca. $2,5 \times 10^4$ $TCID_{50}$ (TCID = tissue culture infectious dosis). Diese Virusdosis entspricht einer MOI (Multiplizität der Infektion) von ca. 0,05.

Unter diesen Infektionsbedingungen resultierte zwischen Tag 5 und Tag 10 in einer Infektionskontrolle ohne Substanz ein virusinduzierter, zytopathischer Effekt. Die infizierten und behandelten Zellen und die Kontrollzellen wurden 7 Tage bei 37°C, 5% $CO_2$ inkubiert.

Bei Auftreten des virusinduzierten zytopathogenen Effektes in der unbehandelten Viruskontrolle wurden die Kulturen mit Formalin fixiert und anschließend mit einer Giemsa-Lösung gefärbt. Die inhibitorische Konzentration ($IC_{50}$) wurde mikroskopisch als die Konzentration ermittelt, bei der der zytopathische Effekt um 50% gehemmt wurde, im Vergleich zur unbehandelten Viruskontrolle, die 100% Zellzerstörung aufwies.

Untersuchungen haben ergeben, daß beispielsweise 3-(1,5-Didesoxy-1,5-imino-D-mannit-N-yl)-propionsäure-4-isopropyl-anilid allein in einem Konzentrationsbereich von 30 µg/ml bis zu 100 µg/ml die mit Visna-Virus infizierten Zellen vor der Virus-induzierten Zellzerstörung schützt.

Schafsinterferon allein, das aus NDV-induzierten Schafszellen gewonnen wurde, zeigte bis zu einer Verdünnung von 1 : 64, entspricht 1 U/ml Interferon, einen Schutzeffekt gegen Visna-Virus-Infektionen. Die Kombination der Verbindung 3-(1,5-Didesoxy-1,5-imino-D-mannit-N-yl)-propionsäure-4- isopropyl-anilid mit Schafsinterferon hingegen führte zu einer starken synergistischen antiviralen Wirksamkeit, die Kombination beider Verbindungen zeigte antivirale Schutzeffekte, die für das Schafsinterferon in einer Verdünnung von 1 : 4096, entspricht 0.02 U/ml Interferon, und für die Verbindung des 3-(1,5-Didesoxy-1,5-imino-D-mannit-N-yl-propionsäure-4-isopropyl-anilid bei einer Konzentration von 1 µg/ml noch zu einem 50%igen Schutzeffekt führten. Das bedeutet, daß durch die Kombination beider Wirkstoffe eine Reduktion der Konzentration der Einzelkomponenten um den Faktor 64 beim Interferon und um den Faktor 30 bei dem 3-(1,5-Didesoxy-1,5-imino-D-mannit-N-yl)-propionsäure-4-isopropyl-anilid möglich ist. Diese Ergebnisse sind in der Tabelle 1 aufgeführt.

**Tabelle 1**

**Zellkultur-Versuch / Behandlung von Schafszellen, die mit Visna-Virus infiziert sind.**

**3-(1,5-Didesoxy-1,5-imino-D-mannit-N-yl)-propionsäure-4-isopropyl-anilid in Kombination mit Interferon**

| Wirkung der einzelnen Verbindungen | | Schafsinterferon (SIF) | | | |
|---|---|---|---|---|---|
| Konzentration der Testsubstanz mg/ml | Antivirale Wirkung in der Zellkultur* | Verdünnung | U/ml | Antivirale Wirkung von Interferon in der Zellkultur | Wirkung der Kombination aus Verbindung und SIF |
| 1 | 3 | 1:8 | 8 | 3 | 3 |
| 0,5 | 3 | 1:16 | 4 | 3 | 3 |
| 0,25 | 3 | 1:32 | 2 | 3 | 3 |
| 0,125 | 3 | 1:64 | 1 | 2 | 3 |
| 0,06 | 3 | 1:128 | 0,5 | 1 | 3 |
| 0,03 | 2 | 1:256 | 0,25 | 0 | 3 |
| 0,015 | 1 | 1:512 | 0,125 | 0 | 3 |
| 0,007 | 0 | 1:1024 | 0,06 | 0 | 3 |
| 0,003 | 0 | 1:2048 | 0,03 | 0 | 2 |
| 0,001 | 0 | 1:4096 | 0,015 | 0 | 2 |

* Bewertung:
3 = kompletter antiviraler Schutzeffekt;
100% Schutz der Zellen vor der Infektion
2 = 50% Schutzeffekt der infizierten Zellkultur
1 = 10 - 20% Schutzeffekt der infizierten Zellkultur
0 = komplette Zerstörung der infizierten Zellkultur

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) in Kombination mit Interferonen enthalten, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Die Kombination der Wirkstoffe kann gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben der Kombination der Wirkstoffe die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben der Kombination der Wirkstoffe die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben der Kombination der Wirkstoffe die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben der Kombination der Wirkstoffe die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die Wirkstoffe der Formel (I) sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den Kombinationen auch weitere

pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramus kulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Kombinationen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, die Kombinationen der Wirkstoffe in Gesamtmengen von 0,5 bis 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält die Kombination der Wirkstoffe vorzugsweise in Mengen von 1 bis 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge der kombinierten Wirkstoffe auszukommen, während in anderen Fällen die oben angeführte kombinierte Wirkstoffmenge überschritten werden muß. Die Festlegung der jeweils erforderlichen optimalen Dosierung und Applikationsart der kombinierten Wirkstoffe kann durch jeden Fachmann aufgrund seines Fachwissens leicht erfolgen.

Die erfindungsgemäß zu verwendenden Kombinationen können in den üblichen Konzentrationen und Zubereitungen zusammen mit dem Futter bzw. mit Futterzubereitungen oder mit dem Trinkwasser gegeben werden.

## Patentansprüche

1. Arzneimittel enthaltend in Kombination Interferon und Verbindungen der allgemeinen Formel (I)

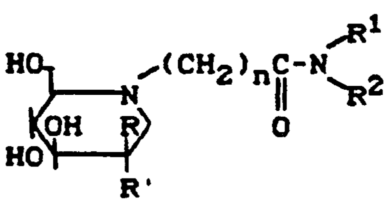

(I)

in welcher

R oder R′ für eine Hydroxylgruppe steht und jeweils andere R′ oder R für Wasserstoff steht,

n —für eine Zahl 2 bis 6 steht,

$R^1$ —für Wasserstoff steht, oder
—für geradkettiges oder verzweigtes Alkyl mit bis zu 3 Kohlenstoffatomen oder für Benzyl steht, und

$R^2$ —für geradkettiges oder verzweigtes Alkyl mit bis zu 8 Kohlenstoffatomen steht, das substituiert sein kann durch Aryl mit 6 bis 10 Kohlenstoffatomen, wobei der Arylrest bis zu 4 gleiche oder verschiedene Substituenten der Reihe Fluor, Chlor, Brom, Iod, Cyano, Nitro, Alkyl, Alkoxy oder Alkythio mit jeweils bis zu 6 Kohlenstoffatomen, Dioxymethylen, Dioxyethylen, Trifluormethyl, Trifluormethoxy, Difluormethoxy, Carboxy oder Alkoxycarbonyl mit bis zu 4 Kohlenstoffatomen tragen kann, oder durch Pyridyl, Thienyl, Furyl, Pyrimidyl, Pyridazinyl, Pyrazinyl oder Chinolyl substituiert sein kann, oder
—für Cycloalkyl mit 3 bis 7 Kohlenstoffatomen steht, oder

—für Aryl mit 6 bis 10 Kohlenstoffatomen steht, wobei der Arylrest bis zu 4-fach gleich oder verschieden substituiert sein kann durch Fluor, Chlor, Brom, Iod, Cyano, Alkyl mit bis zu 6 Kohlenstoffatomen, Alkyloxy mit bis zu 6 Kohlenstoffatomen, Alkylsulfonyl mit jeweils bis zu 4 Kohlenstoffatomen, Trifluormethyl, Trifluormethoxy, Difluormethylen, Difluormethoxy, Trifluormethylthio, Dioxymethylen, Dioxyethylen, durch Cycloalkyl mit 3 bis 7 Kohlenstoffatomen, durch Carboxy oder Alkoxycarbonyl mit bis zu 4 Kohlenstoffatomen, durch Aryloxy mit 6 bis 10 Kohlenstoffatomen, wobei der Arylrest bis zu 4 gleiche oder verschiedene Substituenten der Reihe Fluor, Chlor, Brom, Iod, Cyano, Hydroxy, Nitro, Alkyl, Alkoxy, Alkylthio mit jeweils bis zu 6 Kohlenstoffatomen, Dioxymethylen, Dioxyethylen, Trifluormethyl, Trifluormethoxy oder Difluor methoxy tragen kann, oder durch Halogenalkoxy mit bis zu 6 Kohlenstoffatomen und bis zu 5 Fluor und/oder 3 Chloratomen, oder durch Hydroxyalkoxy, Hydroxyalkyl oder Cyanoalkyl mit jeweils bis zu 8 Kohlenstoffatomen, oder durch Alkenyloxy mit bis zu 6 Kohlenstoffatomen, oder durch eine Gruppe —$XR^3$,
wobei

X —eine geradkettige oder verzweigte Alkylen-oder Alkenylenkette mit bis zu 8 Kohlenstoffatomen bedeutet,

und

$R^3$ —Aryl mit 6 bis 10 Kohlenstoffatomen bedeutet, wobei der Arylrest bis zu 4 gleiche oder verschiedene Substituenten der Reihe Fluor, Chlor, Brom, Iod, Cyano, Alkyl, Alkoxy, Alkylthio mit jeweils bis zu 6 Kohlenstoffatomen, Nitro, Dioxymethylen, Dioxyethylen, Trifluormethyl, Trifluormethoxy, Difluormethoxy oder Trifluormethylthio tragen kann, oder
— einen 5- bis 6-gliedrigen gesättigten oder ungesättigten heterocyclischen Ring bedeutet, der benzokondensiert sein kann und der als Heteroatome Sauerstoff, Schwefel oder bis zu 2 Stickstoffatome enthalten kann, oder
— Hydroxy, Carboxy, Alkoxycarbonyl mit bis zu 6 Kohlenstoffatomen, Alkylcarbonyloxy mit bis zu 18 Kohlenstoffatomen, Cycloalkyl mit 3 bis 6 Kohlenstoffatomen bedeutet, oder
— eine Gruppe —$OSO_3H$, —$CONH$-$NH_2$, —$CONH_2$ oder Amino, Alkylamino oder Dialkylamino mit jeweils bis zu 6 Kohlenstoffatomen je Alkylgruppe bedeutet, oder
– Alkyl oder Hydroxyalkyl mit bis zu 8 Kohlenstoffatomen bedeutet,
oder

$R^1$ und $R^2$ gemeinsam mit dem Stickstoffatom einen 5 bis 6-gliedrigen Ring bilden, der durch Sauerstoff oder die Gruppe —$NR^4$ unterbrochen sein kann, und der substituiert sein kann durch Alkyl mit bis zu 4 Kohlenstoffatomen, Hydroxy, Phenyl, Carboxy oder Alkoxycarbonyl mit bis zu 4 Kohlenstoffatomen,
wobei

$R^4$ — Aryl mit 6 bis 10 Kohlenstoffatomen bedeutet, wobei der Arylrest bis 4 gleiche oder verschiedene Substituenten der Gruppe Fluor, Chlor, Brom, Iod, Cyano, Nitro, Hydroxy, Alkyl, Alkoxy, Alkylthio mit jeweils bis zu 6 Kohlenstoffatomen, Dioxymethylen, Dioxyethylen, Trifluormethyl, Trifluormethoxy oder Difluormethoxy tragen kann, oder
— Alkoxycarbonyl mit bis zu 6 Kohlenstoffatomen bedeutet, oder
— Pyridyl, Pyrimidyl, Furyl, Thienyl, Pyrazinyl, Pyridazinyl oder Chinolyl bedeutet, oder
— Cycloalkyl mit 3 bis 7 Kohlenstoffatomen bedeutet, oder
—Alkyl oder Alkenyl mit bis zu 6 Kohlenstoffatomen bedeutet, das bis zu 2-fach gleich oder verschieden substituiert sein kann durch gegebenenfalls durch Chlor, Dioxymethylen oder Trifluormethyl substituiertes Phenyl, Hydroxy, Amino, Alkylamino, Dialkylamino mit jeweils bis zu 3 Kohlenstoffatomen je Alkylgruppe, Fluor, Chlor, Brom, Cycloalkyl mit 3 bis 6 Kohlenstoffatomen, durch Carboxy oder Alkoxycarbonyl mit bis zu 6 Kohlenstoffatomen, durch Pyridyl, Pyrimidyl, Pyrazinyl, Morpholinyl, Thiomorpholinyl oder Pyrrolidinyl, durch Morpholinocarbonyl, durch Alkylaminocarbonyl, Dialkylaminocarbonyl oder Phenylalkylaminocarbonyl mit jeweils bis zu 6 Kohlenstoffatomen je Alkylgruppe.

2. Arzneimittel enthaltend in Kombination Interferon und Verbindungen der allgemeinen Formel (I) gemäß Anspruch 1,
in welcher

n —für die Zahl 2 bis 4 steht,

$R^1$ — für Wasserstoff steht,
und

$R^2$ — für geradkettiges oder verzweigtes Alkyl mit bis zu 6 Kohlenstoffatomen steht, das substituiert sein kann durch Pyridyl, oder durch Phenyl, wobei der Phenylrest bis zu 3 gleiche oder verschiedene Substituenten der Reihe Fluor, Chlor, Brom, Hydroxy, Alkyl, Alkoxy mit bis zu 4 Kohlenstoffatomen, Dioxymethylen, Dioxyethylen oder Carboxy tragen kann, oder
— für Cyclopentyl, Cyclohexyl oder Cycloheptyl steht, oder
— für Phenyl steht, das bis zu 3-fach gleich oder verschieden substituiert sein kann durch Fluor, Chlor, Brom, Cyano, Alkyl mit bis zu 4 Kohlenstoffatomen, Hydroxyalkyl oder Hydroxyalkoxy mit bis zu 4 Kohlenstoffatomen, Trifluormethyl, Trifluormethoxy, Dioxymethylen, Dioxyethylen, durch gegebenenfalls im Aromaten durch Fluor, Chlor, Brom, Hydroxy, Alkyl, Alkoxy mit bis zu 4 Kohlenstoffatomen, Dioxymethylen, Trifluormethyl oder Carboxy substituiertes Phenoxy, oder durch geradkettiges oder verzweigtes Alkenyloxy mit bis zu 4 Kohlenstoffatomen, oder durch eine Gruppe —$XR^3$
wobei

X — eine geradkettige oder verzweigte Alkylenkette mit bis zu 6 Kohlenstoffatomen bedeutet,
und

$R^3$ ein bis zu 3-fach gleich oder verschieden durch Fluor, Chlor, Brom, Hydroxy, Alkyl, Alkoxy mit bis zu 4 Kohlenstoffatomen, Dioxymethylen, Trifluormethyl oder Carboxy substituiertem Phenyl bedeutet, oder
— Pyridyl, Morpholinyl oder Piperidinyl, bedeutet, oder
— Hydroxy, Carboxy, Alkoxycarbonyl mit bis zu 4 Kohlenstoffatomen, oder Cyclopropyl, Cyclopentyl oder Cyclohexyl bedeutet, oder
— Amino, Alkylamino oder Dialkylamino mit jeweils bis zu 4 Kohlenstoffatomen je Alkylgruppe bedeutet,

oder

$R^1$ und $R^2$ gemeinsam mit dem Stickstoffatom einen Ring der Formel

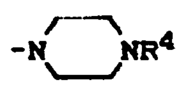 , 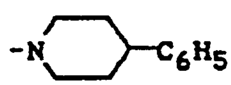 , 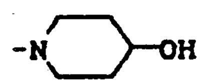 ,

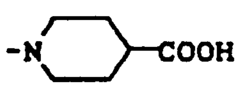 oder 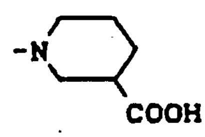

bilden, wobei

$R^4$ — einen gegebenenfalls bis zu 3-fach gleich oder verschieden durch Fluor, Chlor, Brom, Hydroxy, Alkyl, Alkoxy mit bis zu 4 Kohlenstoffatomen, Dioxymethylen, Trifluormethyl oder Carboxy substituiertes Phenyl bedeutet, oder
— Alkoxycarbonyl mit bis zu 4 Kohlenstoffatomen bedeutet, oder
— Pyridyl oder Pyrimidyl bedeutet, oder
— Cyclopropyl, Cyclopentyl oder Cyclohexyl bedeutet, oder
— geradkettiges oder verzweigtes Alkyl oder Alkenyl mit bis zu 4 Kohlenstoffatomen bedeutet, das bis zu 2-fach gleich oder verschieden durch gegebenenfalls durch Chlor, Trifluormethyl oder Dioxymethylen substituiertes Phenyl, Hydroxy, Amino, Methylamino, Dimethylamino, Fluor, Chlor, Cyclopropyl, Cyclopentyl, Cyclohexyl oder Alkoxycarbonyl mit bis zu 4 Kohlenstoffatomen, durch Morpholinyl, Pyrrolidinyl, Morpholinocarbonyl, Dialkylamino oder Phenylalkylamino mit jeweils bis zu 4 Kohlenstoffatomen substituiert sein kann.

3. Arzneimittel enthaltend in Kombination Interferon und Verbindungen der allgemeinen Formel (I) gemäß Anspruch 1,

in welcher

n — für die Zahl 2 steht,

$R^1$ — für Wasserstoff steht, und

$R^2$ — für Phenyl steht, das bis zu 3-fach gleich oder verschieden substituiert sein kann durch Fluor, Chlor, Brom, Cyano, Alkyl mit bis zu 4 Kohlenstoffatomen, Hydroxyalkyl, Hydroxyalkoxy mit bis zu 4 Kohlenstoffatomen, Alkylthio mit bis zu 4 Kohlenstoffatomen, Trifluormethyl, Trifluormethoxy, oder

$R^1$ und $R^2$ gemeinsam mit dem Stickstoff einen Ring der Formel

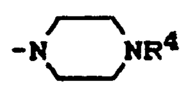 bilden,

wobei

$R^4$ — einen gegebenenfalls bis zu 2-fach gleich oder verschieden durch Fluor, Chlor, Brom, Hydroxy, Alkyl, Alkoxy mit bis zu 4 Kohlenstoffatomen, substituiertes Phenyl bedeutet, oder
— Alkoxycarbonyl mit bis zu 4 Kohlenstoffatomen bedeutet, oder
— Cyclopropyl, Cyclopentyl oder Cyclohexyl bedeutet, oder
— geradkettiges oder verzweigtes Alkyl oder Alkenyl mit bis zu 4 Kohlenstoffatomen bedeutet, das bis zu 2-fach gleich oder verschieden durch gegebenenfalls durch Chlor, Trifluormethyl, oder Dioxymethylen substituiertes Phenyl, Hydroxy, Amino, Dimethylamino, Cyclopropyl oder Alkoxycarbonyl mit bis zu 4 Kohlenstoffatomen, durch Morpholinyl oder Pyrrolidinyl substituiert sein kann.

4. Arneimittel enthaltend in Kombination Interferon und Verbindungen aus der Gruppe
3-(1,5-Didesoxy-1,5-imino-D-glucit-N-yl)-propionsäure-(4-methoxy)-anilid,
3-(1,5-Didesoxy-1,5-imino-D-glucit-N-yl)-propionsäure-(4-methoxycarbonyl)-anilid,
3-(1,5-Didesoxy-1,5-imino-D-glucit-N-yl)-propionsäure-4-(4-pyridylmethoxy)-anilid,
3-(1,5-Didesoxy-1,5-imino-D-glucit-N-yl)-propionsäure-4-benzyloxy-anilid,
3-(1,5-Didesoxy-1,5-imino-D-glucit-N-yl)-propionsäure-4-trifluormethoxy-anilid,
3-(1,5-Didesoxy-1,5-imino-D-glucit-N-yl)-propionsäure-4-(2-oxocyclohexyloxy)-anilid,
3-(1,5-Didesoxy-1,5-imino-D-glucit-N-yl)-propionsäure-4-tert.butyl-anilid,
3-(1,5-Didesoxy-1,5-imino-D-glucit-N-yl)-propionsäure-4-(6-methyl-heptyloxy)-anilid,
3-(1,5-Didesoxy-1,5-imino-D-glucit-N-yl)-propionsäure-4-(2-N,N-diethyl-amino-ethoxy)-anilid,
3-(1,5-Didesoxy-1,5-imino-D-glucit-N-yl)-propionsäure-4-allyloxy-anilid,
3-(1,5-Didesoxy-1,5-imino-D-glucit-N-yl)-propionsäure-4-trifluormethyl-anilid,
3-(1,5-Didesoxy-1,5-imino-D-glucit-N-yl)-propionsäure-4-(2-chlor-1,1,2-trifluorethoxy)-anilid,
3-(1,5-Didesoxy-1,5-imino-D-glucit-N-yl)-propionsäure-4-isopropyl-anilid,
3-(1,5-Didesoxy-1,5-imino-D-glucit-N-yl)-propionsäure-2-isopropyl-anilid,
3-(1,5-Didesoxy-1,5-imino-D-glucit-N-yl)-propionsäure-4-fluor-anilid,
3-(1,5-Didesoxy-1,5-imino-D-glucit-N-yl)-propionsäure-2-fluor-anilid,
1-[3-(1,5-Didesoxy-1,5-imino-D-glucit-N-yl)-propionyl]-2,3-dihydroindol,
3-(1,5-Didesoxy-1,5-imino-D-glucit-N-yl)-propionsäure-2,4-difluoranilid,
3-(1,5-Didesoxy-1,5-imino-D-glucit-N-yl)-propionsäure-2-trifluormethyl-anilid,
3-(1,5-Didesoxy-1,5-imino-D-glucit-N-yl)-propionsäure-2,6-difluoranilid,
3-(1,5-Didesoxy-1,5-imino-D-glucit-N-yl)-propionsäure-(adamantan-1-yl)-amid,
3-(1,5-Didesoxy-1,5-imino-D-glucit-N-yl)-propionsäure-3,4,5-trimethoxy-anilid,
3-(1,5-Didesoxy-1,5-imino-D-glucit-N-yl)-propionsäure-3,4-dimethoxy-anilid,
3-(1,5-Didesoxy-1,5-imino-D-glucit-N-yl)-propionsäure-3,5-dimethoxy-anilid,
3-(1,5-Didesoxy-1,5-imino-D-glucit-N-yl)-propionsäure-2,4,6-tribromanilid,
3-(1,5-Didesoxy-1,5-imino-D-glucit-N-yl)-propionsäure-3-cyano-anilid,
3-(1,5-Didesoxy-1,5-imino-D-glucit-N-yl)-propionsäure-4-cyclohexyl-anilid,
3-(1,5-Didesoxy-1,5-imino-D-glucit-N-yl)-propionsäure-3,4-ethylendioxy-anilid,

3-(1,5-Didesoxy-1,5-imino-D-glucit-N-yl)-propionsäure-2-bromanilid,
3-(1,5-Didesoxy-1,5-imino-D-glucit-N-yl)-propionsäure-3,4-methylendioxy-anilid,
3-(1,5-Didesoxy-1,5-imino-D-glucit-N-yl)-propionsäure-2-methylthio-anilid,
3-(1,5-Didesoxy-1,5-imino-D-glucit-N-yl)-propionyl-4-(4-trifluormethyl-phenyl)- piperazin,
3-(1,5-Didesoxy-1,5-imino-D-glucit-N-yl)-propionsäure-anilid,
3-(1,5-Didesoxy-1,5-imino-D-glucit-N-yl)-propionsäure-4-ethoxy-anilid,
3-(1,5-Didesoxy-1,5-imino-D-glucit-N-yl)-propionsäure-2-dimethylaminoethyl-amid,
3-(1,5-Didesoxy-1,5-imino-D-glucit-N-yl)-4-(2-hydroxyethoxy)-anilid,
3-(1,5-Didesoxy-1,5-imino-D-glucit-N-yl)-propionsäure-[2-(3-methoxyphenyl)ethyl)-amid,
3-(1,5-Didesoxy-1,5-imino-D-glucit-N-yl)-propionsäure-2-pyridylmethyl-amid,
3-(1,5-Didesoxy-1,5-imino-D-glucit-N-yl)-propionyl-(4-cinnamyl)-piperazin,
säure-(4-methoxy)-anilid,
3-(1,5-Didesoxy-1,5-imino-D-mannit-N-yl)-propionsäure-(4-methoxycarbonyl)-anilid,
3-(1,5-Didesoxy-1,5-imino-D-mannit-N-yl)-propionsäure-4-(4-pyridylmethoxy)-anilid,
3-(1,5-Didesoxy-1,5-imino-D-mannit-N-yl)-propionsäure-4-benzyloxy-anilid,
3-(1,5-Didesoxy-1,5-imino-D-mannit-N-yl)-propionsäure-4-trifluormethoxy-anilid,
3-(1,5-Didesoxy-1,5-imino-D-mannit-N-yl)-propionsäure-4-(2-oxocyclohexyloxy)-anilid,
3-(1,5-Didesoxy-1,5-imino-D-mannit-N-yl)-propionsäure-4-tert.butyl-anilid,
3-(1,5-Didesoxy-1,5-imino-D-mannit-N-yl)-propionsäure-4-(6-methyl-heptyloxy)-anilid,
3-(1,5-Didesoxy-1,5-imino-D-mannit-N-yl)-propionsäure-4-(2-N,N-diethyl-amino-ethoxy)-anilid,
3-(1,5-Didesoxy-1,5-imino-D-mannit-N-yl)-propionsäure-4-allyloxy-anilid,
3-(1,5-Didesoxy-1,5-imino-D-mannit-N-yl)-propionsäure-4-trifluormethyl-anilid,
3-(1,5-Didesoxy-1,5-imino-D-mannit-N-yl)-propionsäure-4-(2-chlor-1,1,2-trifluorethoxy)-anilid,
3-(1,5-Didesoxy-1,5-imino-D-mannit-N-yl)-propionsäure-4-isopropyl-anilid,
3-(1,5-Didesoxy-1,5-imino-D-mannit-N-yl)-propionsäure-2-isopropyl-anilid,
3-(1,5-Didesoxy-1,5-imino-D-mannit-N-yl)-propionsäure-4-fluor-anilid,
3-(1,5-Didesoxy-1,5-imino-D-mannit-N-yl)-propionsäure-2-fluor-anilid,
1-[3-(1,5-Didesoxy-1,5-imino-D-mannit-N-yl)-propionyl]-2,3-dihydroindol,
3-(1,5-Didesoxy-1,5-imino-D-mannit-N-yl)-propionsäure-2,4-difluoranilid,
3-(1,5-Didesoxy-1,5-imino-D-mannit-N-yl)-propionsäure-2-trifluormethyl-anilid,
3-(1,5-Didesoxy-1,5-imino-D-mannit-N-yl)-propionsäure-2,6-difluoranilid,
3-(1,5-Didesoxy-1,5-imino-D-mannit-N-yl)-propionsäure-(adamantan-1-yl)-amid,
3-(1,5-Didesoxy-1,5-imino-D-mannit-N-yl)-propionsäure-3,4,5-trimethoxy-anilid,
3-(1,5-Didesoxy-1,5-imino-D-mannit-N-yl)-propionsäure-3,4-dimethoxy-anilid,
3-(1,5-Didesoxy-1,5-imino-D-mannit-N-yl)-propionsäure-3,5-dimethoxy-anilid,
3-(1,5-Didesoxy-1,5-imino-D-mannit-N-yl)-propionsäure-2,4,6-tribromanilid,
3-(1,5-Didesoxy-1,5-imino-D-mannit-N-yl)-propionsäure-3-cyano-anilid,
3-(1,5-Didesoxy-1,5-imino-D-mannit-N-yl)-propionsäure-4-cyclohexyl-anilid,
3-(1,5-Didesoxy-1,5-imino-D-mannit-N-yl)-propionsäure-3,4-ethylendioxy-anilid,
3-(1,5-Didesoxy-1,5-imino-D-mannit-N-yl)-propionsäure-2-bromanilid,
3-(1,5-Didesoxy-1,5-imino-D-mannit-N-yl)-propionsäure-3,4-methylendioxy-anilid,
3-(1,5-Didesoxy-1,5-imino-D-mannit-N-yl)-propionsäure-2-methylthio-anilid,
3-(1,5-Didesoxy-1,5-imino-D-mannit-N-yl)-propionyl-4-(4-trifluormethyl-phenyl)- piperazin,
3-(1,5-Didesoxy-1,5-imino-D-mannit-N-yl)-propionsäure-anilid,
3-(1,5-Didesoxy-1,5-imino-D-mannit-N-yl)-propionsäure-4-ethoxy-anilid,
3-(1,5-Didesoxy-1,5-imino-D-mannit-N-yl)-propionsäure-2-dimethylaminoethyl-amid,
3-(1,5-Didesoxy-1,5-imino-D-mannit-N-yl)-propionsäure-[2-(3-methoxyphenyl)ethyl)-amid,
3-(1,5-Didesoxy-1,5-imino-D-mannit-N-yl)-propionsäure-2-pyridylmethyl-amid,
3-(1,5-Didesoxy-1,5-imino-D-mannit-N-yl)-propionyl-(4-cinnamyl)-piperazin und
3-(1,5-Didesoxy-1,5-imino-D-mannit-N-yl)-4-(2-hydroxyethoxy)-anilid.

5. Arzneimittel gemäß Ansprüchen 1 bis 4 zur Bekämpfung von viralen Erkrankungen.

6. Arzneimittel gemäß den Ansprüchen 1 bis 4 zur Bekämpfung von retroviralen Erkrankungen.

7. Arzneimittel gemäß den Ansprüchen 1 bis 4 zur Bekämpfung von HIV Infektionen.

## Claims

1. Medicaments containing, in combination, interferon and compounds of the general formula (I)

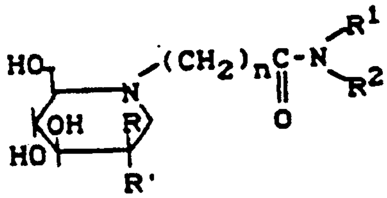

in which

R or R′ represents a hydroxyl group and the other R′ or R in each case represents hydrogen,
n — represents a number from 2 to 6,
$R^1$ — represents hydrogen, or
— represents straight-chain or branched alkyl having up to 3 carbon atoms or benzyl,
and

$R^2$ — represents straight-chain or branched alkyl having up to 8 carbon atoms, which can be substituted by aryl having 6 to 10 carbon atoms, where the aryl radical can carry up to 4 identical or different substituents from the series comprising fluorine, chlorine, bromine, iodine, cyano, nitro, alkyl, alkoxy or alkylthio each having up to 6 carbon atoms, dioxymethylene, dioxyethylene, trifluoromethyl, trifluoromethoxy, difluoromethoxy, carboxyl or alkoxycarbonyl having up to 4 carbon atoms, or can be substituted by pyridyl, thienyl, furyl, pyrimidyl, pyridazinyl, pyrazinyl or quinolyl, or
— represents cycloalkyl having 3 to 7 carbon atoms, or
— represents aryl having 6 to 10 carbon atoms, where the aryl radical can be monosubstituted to tetrasubstituted by identical or different substituents from the series comprising fluorine, chlorine, bromine, iodine, cyano, alkyl having up to 6 carbon atoms, alkyloxy having up to 6 carbon atoms, alkylsulphonyl each having up to 4 carbon atoms, trifluoromethyl, trifluoromethoxy, difluoromethylene, difluoromethoxy, trifluoromethylthio, dioxymethylene, dioxyethylene, by cycloalkyl having 3 to 7 carbon atoms, by carboxyl or alkoxycarbonyl having up to 4 carbon atoms or by aryloxy having 6 to 10 carbon atoms, where the aryl radical can carry up to 4 identical or different substituents from the series comprising fluorine, chlorine, bromine, iodine, cyano, hydroxyl, nitro, alkyl, alkoxy or alkylthio each having up to 6 carbon atoms, dioxymethylene, dioxyethylene, trifluoromethyl, trifluoromethoxy or difluoromethoxy, or by halogenoalkoxy having up to 6 carbon atoms and up to 5 fluorine and/or 3 chlorine atoms, or by hydroxyalkoxy, hydroxyalkyl or cyanoalkyl each having up to 8 carbon atoms, or by alkenyloxy having up to 6 carbon atoms, or by a group —$XR^3$,
where

X — denotes a straight-chain or branched alkylene or alkenylene chain having up to 8 carbon atoms,
and

$R^3$ — denotes aryl having 6 to 10 carbon atoms, where the aryl radical can carry up to 4 identical or different substituents from the series comprising fluorine, chlorine, bromine, iodine, cyano, alkyl, alkoxy or alkylthio each having up to 6 carbon atoms, nitro, dioxymethylene, dioxyethylene, tryfluoromethyl, trifluoromethoxy, difluoromethoxy or trifluoromethylthio, or
— denotes a 5- to 6-membered saturated or unsaturated heterocyclic ring which can be fused to benzene and which can contain oxygen, sulphur or up to 2 nitrogen atoms as heteroatoms, or
— denotes hydroxyl, carboxyl, alkoxycarbonyl having up to 6 carbon atoms, alkylcarbonyloxy having up to 18 carbon atoms, cycloalkyl having 3 to 6 carbon atoms, or
— denotes a group —$OSO_3H$, —$CONH\text{-}NH_2$, —$CONH_2$ or amino, alkylamino or dialkylamino each having up to 6 carbon atoms per alkyl group, or
— denotes alkyl or hydroxyalkyl having up to 8 carbon atoms,
or

$R^1$ and $R^2$, together with the nitrogen atom, form a 5- to 6-membered ring which can be interrupted by oxygen or the group —$NR^4$, and which can be substituted by alkyl having up to 4 carbon atoms, hydroxyl, phenyl, carboxyl or alkoxycarbonyl having up to 4 carbon atoms,
where

$R^4$ — denotes aryl having 6 to 10 carbon atoms, where the aryl radical can carry up to 4 identical or different substituents from the group comprising fluorine, chlorine, bromine, iodine, cyano, nitro, hydroxyl, alkyl, alkoxy or alkylthio each having up to 6 carbon atoms, dioxymethylene, dioxyethylene, trifluoromethyl, trifluoromethoxy or difluoromethoxy, or
— denotes alkoxycarbonyl having up to 6 carbon atoms, or
— denotes pyridyl, pyrimidyl, furyl, thienyl, pyrazinyl, pyridazinyl or quinolyl, or
— denotes cycloalkyl having 3 to 7 carbon atoms, or
— denotes alkyl or alkenyl having up to 6 carbon atoms, which can be monosubstituted or disubstituted by identical or different phenyl which is optionally substituted by chlorine, dioxymethylene or trifluoromethyl, hydroxyl, amino, alkylamino or dialkylamino each having up to 3 carbon atoms per alkyl group, fluorine, chlorine, bromine, cycloalkyl having 3 to 6 carbon atoms, by carboxyl or alkoxycarbonyl having up to 6 carbon atoms, by pyridyl, pyrimidyl, pyrazinyl, morpholinyl, thiomorpholinyl or pyrrolidinyl, by morpholinocarbonyl, by alkylaminocarbonyl, dialkylaminocarbonyl or phenylalkylaminocarbonyl each having up to 6 carbon atoms per alkyl group.

2. Medicaments containing, in combination, interferon and compounds of the general formula (1) according to Claim 1,
in which

n — represents the numbers 2 to 4,
$R^1$ — represents hydrogen,
and

$R^2$ — represents straight-chain or branched alkyl having up to 6 carbon atoms, which can be substituted by pyridyl, or by phenyl, where the phenyl radical can carry up to 3 identical or different substituents from the series comprising fluorine, chlorine, bromine, hydroxyl, alkyl, alkoxy having up to 4 carbon atoms, dioxymethylene, dioxyethylene or carboxyl, or
— represents cyclopentyl, cyclohexyl or cycloheptyl, or
— represents phenyl which can be monosubstituted, disubstituted or trisubstituted by identical or different fluorine, chlorine, bromine, cyano, alkyl having up to 4 carbon atoms, hydroxyalkyl or hydroxyalkoxy having up to 4 carbon atoms, trifluoromethyl, trifluoromethoxy, dioxymethylene, dioxyethylene, by phenoxy which is optionally substituted in the aromatic moiety by fluorine, chlorine, bromine, hydroxyl, alkyl, alkoxy having up to 4 carbon atoms, dioxymethylene, trifluoromethyl or carboxyl, or by straight-chain or branched alkenyloxy having up to 4 carbon atoms, or by a group $—XR^3$
where

X — denotes a straight-chain or branched alkylene chain having up to 6 carbon atoms
and

$R^3$ — denotes phenyl which is monosubstituted, disubstituted or trisubstituted by identical or different fluorine, chlorine, bromine, hydroxyl, alkyl, alkoxy having up to 4 carbon atoms, dioxymethylene, trifluoromethyl or carboxyl, or
— denotes pyridyl, morpholinyl or piperidinyl, or
— denotes hydroxyl, carboxyl, alkoxycarbonyl having up to 4 carbon atoms, or cyclopropyl, cyclopentyl or cyclohexyl, or
— denotes amino, alkylamino or dialkylamino each having up to 4 carbon atoms per alkyl group,
or

$R^1$ and $R^2$ , together with the nitrogen, form a ring of the formula

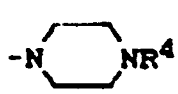 , 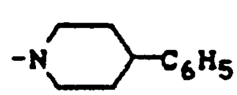 , 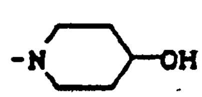

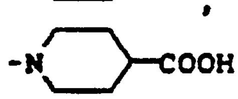 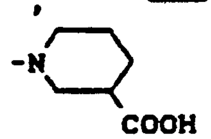

where

$R^4$ — denotes phenyl which is optionally monosubstituted, disubstituted or trisubstituted by identical or different fluorine, chlorine, bromine, hydroxyl, alkyl, alkoxy having up to 4 carbon atoms, dioxymethylene, trifluoromethyl or carboxyl, or
— denotes alkoxycarbonyl having up to 4 carbon atoms, or
— denotes pyridyl or pyrimidyl, or
— denotes cyclopropyl, cyclopentyl or cyclohexyl, or
— denotes straight-chain or branched alkyl or alkenyl having up to 4 carbon atoms, which can be monosubstituted or disubstituted by identical or different phenyl which is optionally substituted by chlorine, trifluoromethyl or dioxymethylene, hydroxyl, amino, methylamino, dimethylamino, fluorine, chlorine,cyclopropyl, cyclopentyl, cyclohexyl, or alkoxycarbonyl having up to 4 carbon atoms, by morpholinyl, pyrrolidinyl, morpholinocarbonyl, dialkylamino or phenylalkylamino each having up to 4 carbon atoms.

3. Medicaments containing, in combination, interferon and compounds of the general formula (I) according to Claim 1,
in which

n — represents the number 2,
$R^1$ — represents hydrogen,
and

$R^2$ — represents phenyl which can be monosubstituted, disubstituted or trisubstituted by identical or different fluorine, chlorine, bromine, cyano, alkyl having up to 4 carbon atoms, hydroxyalkyl, hydroxyalkoxy having up to 4 carbon atoms, alkylthio having up to 4 carbon atoms, trifluoromethyl or trifluoromethoxy,

or

$R^1$ and $R^2$ , together with nitrogen, form a ring of the formula

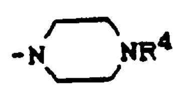

where

$R^4$ — denotes phenyl which is optionally monosubstituted or disubstituted by identical or different fluorine, chlorine, bromine, hydroxyl, alkyl or alkoxy having up to 4 carbon atoms, or
— denotes alkoxycarbonyl having up to 4 carbon atoms, or
— denotes cyclopropyl, cyclopentyl or cyclohexyl, or
— denotes straight-chain or branched alkyl or alkenyl having up to 4 carbon atoms, which can be monosubstituted or disubstituted by identical or different substituents from the series comprising phenyl which is optionally substituted by chlorine, trifluoromethyl or dioxymethylene, hydroxyl, amino, dimethylamino, cyclopropyl or alkoxycarbonyl having up to 4 carbon atoms, by morpholinyl or pyrrolidinyl.

4. Medicaments containing, in combination, interferon and compounds fron the group comprising
N-(4-methoxyphenyl)-3-(1,5-didesoxy-1,5-imino-D-glucit-N-yl)propionamide,
N-(4-methoxycarbonylphenyl)-3-(1,5-didesoxy-1,5-imino-D-glucit-N-yl)propionamide,
N-[4-(4-pyridylmethoxyphenyl)]-3-(1,5-didesoxy-1,5-imino-D-glucit-N-yl)propionamide,
N-(4-benzyloxyphenyl)-3-(1,5-didesoxy-1,5-imino-D-glucit-N-yl)propionamide,
N-(4-trifluoromethoxyphenyl)-3-(1,5-didesoxy-1,5-imino-D-glucit-N-yl)propionamide,
N-[4-(2-oxocyclohexyloxy)phenyl]-3-(1,5-didesoxy-1,5-imino-D-glucit-N-yl)propionamide,
N-(4-tert.-butylphenyl)-3-(1,5-didesoxy-1,5-imino-D-glucit-N-yl)propionamide,
N-[4-(6-methylheptyloxy)phenyl]-3-(1,5-didesoxy-1,5-imino-D-glucit-N-yl)propionamide,
N-[4-(2-N,N-diethylaminoethoxy)phenyl]-3-(1,5-didesoxy-1,5-imino-D-glucit-N-yl)propionamide,
N-(4-allyloxyphenyl)-3-(1,5-didesoxy-1,5-imino-D-glucit-N-yl)propionamide,

N-(4-trifluoromethylphenyl)-3-(1,5-didesoxy-1,5-imino-D-glucit-N-yl)propionamide,
N-[4-(2-chloro-1,1,2-trifluoroethoxy)phenyl]-3-(1,5-didesoxy-1,5-imino-D-glucit-N-yl)propionamide,
N-(4-isopropylphenyl)-3-(1,5-didesoxy-1,5-imino-D-glucit-N-yl)propionamide,
N-(2-isopropylphenyl)-(1,5-didesoxy-1,5-imino-D-glucit-N-yl)propionamide,
N-(4-fluorophenyl)-3-(1,5-didesoxy-1,5-imino-D-glucit-N-yl)propionamide,
N-(2-fluorophenyl)-3-(1,5-didesoxy-1,5-imino-D-glucit-N-yl)propionamide,
1-[3-(1,5-didesoxy-1,5-imino-D-glucit-N-yl)-propionyl]-2,3-dihydroindole,
N-(2,4-difluorophenyl)-3-(1,5-didesoxy-1,5-imino-D-glucit-N-yl)propionamide,
N-(2-trifluoromethylphenyl)-3-(1,5-didesoxy-1,5-imino-D-glucit-N-yl)propionamide,
N-(2,6-difluorophenyl)-3-(1,5-didesoxy-1,5-imino-D-glucit-N-yl)propionamide,
N-(adamantan-1-yl)-3-(1,5-didesoxy-1,5-imino-D-glucit-N-yl)propionamide,
N-(3,4,5-trimethoxyphenyl)-3-(1,5-didesoxy-1,5-imino-D-glucit-N-yl)propionamide,
N-(3,4-dimethoxyphenyl)-3-(1,5-didesoxy-1,5-imino-D-glucit-N-yl)propionamide,
N-(3,5-dimethophenyl)-3-(1,5-didesoxy-1,5-imino-D-glucit-N-yl)propionamide,
N-(2,4,6-tribromophenyl)-3-(1,5-didesoxy-1,5-imino-D-glucit-N-yl)propionamide,
N-(3-cyanophenyl)-3-(1,5-didesoxy-1,5-imino-D-glucit-N-yl)propionamide,
N-(4-cyclohexylphenyl)-3-(1,5-didesoxy-1,5-imino-D-glucit-N-yl)propionamide,
N-(3,4-ethylenedioxyphenyl)-3-(1,5-didesoxy-1,5-imino-D-glucit-N-yl)propionamide,
N-(2-bromophenyl)-3-(1,5-didesoxy-1,5-imino-D-glucit-N-yl)propionamide,
N-(3,4-methylenedioxyphenyl)-3-(1,5-didesoxy-1,5-imino-D-glucit-N-yl)propionamide,
N-(2-methylthiophenyl)-3-(1,5-didesoxy-1,5-imino-D-glucit-N-yl)propionamide,
3-(1,5-didesoxy-1,5-imino-D-glucit-N-yl)-propionyl-4-(4-trifluoromethylphenyl)piperazine,
N-phenyl-3-(1,5-didesoxy-1,5-imino-D-glucit-N-yl)-propionamide,
N-(4-ethoxyphenyl)-3-(1,5-didesoxy-1,5-imino-D-glucit-N-yl)propionamide,
N-(2-dimethylaminoethyl)-3-(1,5-didesoxy-1,5-imino-D-glucit-N-yl)propionamide,
N-4-(2-hydroxyethoxy)phenyl-3-(1,5-didesoxy-1,5-imino-D-glucit-N-yl)propionamide,
N-[2-(3-methoxyphenyl)ethyl]-3-(1,5-didesoxy-1,5-imino-D-glucit-N-yl)propionamide,
N-(2-pyridylmethyl)-3-(1,5-didesoxy-1,5-imino-D-glucit-N-yl)propionamide,
3-(1,5-didesoxy-1,5-imino-D-glucit-N-yl)-propionyl-(4-cinnamyl)piperazine,
N-(4-methoxyphenyl)-3-(1,5-didesoxy-1,5-imino-D-mannit-N-yl)propionamide,
N-(4-methoxycarbonylphenyl)-3-(1,5-didesoxy-1,5-imino-D-mannit-N-yl)propionamide,
N-[4-(4-pyridylmethoxy)phenyl]-3-(1,5-didesoxy-1,5-imino-D-mannit-N-yl)propionamide,
N-(4-benzyloxyphenyl)-3-(1,5-didesoxy-1,5-imino-D-mannit-N-yl)propionamide,
N-(4-trifluoromethoxyphenyl)-3-(1,5-didesoxy-1,5-imino-D-mannit-N-yl)propionamide,
N-[4-(2-oxocyclohexyloxy)phenyl]-3-(1,5-didesoxy-1,5-imino-D-mannit-N-yl)propionamide,
N-(4-tert.-butylphenyl)-3-(1,5-didesoxy-1,5-imino-D-mannit-N-yl)propionamide,
N-[4-(6-methylheptyloxy)phenyl]-3-(1,5-didesoxy-1,5-imino-D-mannit-N-yl)propionamide,
N-[4-(2-N,N-diethylaminoethoxy)phenyl]-3-(1,5-didesoxy-1,5-imino-D-mannit-N-yl)propionamide,
N-(4-allyloxyphenyl)-3-(1,5-didesoxy-1,5-imino-D-mannit-N-yl)propionamide,
N-(4-trifluoromethylphenyl)-3-(1,5-didesoxy-1,5-imino-D-mannit-N-yl)propionamide,
N-[4-(2-chloro-1,1,2-trifluoroethoxy)phenyl]-3-(1,5-didesoxy-1,5-imino-D-mannit-N-yl)propionamide,
N-(4-isopropylphenyl)-3-(1,5-didesoxy-1,5-imino-D-mannit-N-yl)propionamide,
N-(2-isopropylphenyl)-3-(1,5-didesoxy-1,5-imino-D-mannit-N-yl)propionamide,
N-(4-fluorophenyl)-3-(1,5-didesoxy-1,5-imino-D-mannit-N-yl)propionamide,
N-(2-fluorophenyl)-3-(1,5-didesoxy-1,5-imino-D-mannit-N-yl)propionamide,
1-[3-(1,5-didesoxy-1,5-imino-D-mannit-N-yl)-propionyl]-2,3-dihydroindole,
N-(2,4-difluorophenyl)-3-(1,5-didesoxy-1,5-imino-D-mannit-N-yl)propionamide,
N-(2-trifluoromethylphenyl)-3-(1,5-didesoxy-1,5-imino-D-mannit-N-yl)propionamide,
N-(2,6-difluorophenyl)-3-(1,5-didesoxy-1,5-imino-D-mannit-N-yl)propionamide,
N-(adamantan-1-yl)-3-(1,5-didesoxy-1,5-imino-D-mannit-N-yl)propionamide,
N-(3,4,5-trimethoxyphenyl)-3-(1,5-didesoxy-1,5-imino-D-mannit-N-yl)propionamide,
N-(3,4-dimethoxyphenyl)-3-(1,5-didesoxy-1,5-imino-D-mannit-N-yl)propionamide,
N-(3,5-dimethoxyphenyl)-3-(1,5-didesoxy-1,5-imino-D-mannit-N-yl)propionamide,
N-(2,4,6-tribromophenyl)-3-(1,5-didesoxy-1,5-imino-D-mannit-N-yl)propionamide,
N-(3-cyanophenyl)-3-(1,5-didesoxy-1,5-imino-D-mannit-N-yl)propionamide,
N-(4-cyclohexylphenyl)-3-(1,5-didesoxy-1,5-imino-D-mannit-N-yl)propionamide,
N-(3,4-ethylenedioxyphenyl)-3-(1,5-didesoxy-1,5-imino-D-mannit-N-yl)propionamide,
N-(2-bromophenyl)-3-(1,5-didesoxy-1,5-imino-D-mannit-N-yl)propionamide,

N-(3,4-methylenedioxyphenyl)-3-(1,5-didesoxy-1,5-imino-D-mannit-N-yl)propionamide,
N-(2-methylthiophenyl)-3-(1,5-didesoxy-1,5-imino-D-mannit-N-yl)propionamide,
3-(1,5-didesoxy-1,5-imino-D-mannit-N-yl)-propionyl-4-(4-trifluoromethylphenyl)piperazine,
N-phenyl-3-(1,5-didesoxy-1,5-imino-D-mannit-N-yl)propionamide,
N-(4-ethoxyphenyl)-3-(1,5-didesoxy-1,5-imino-D-mannit-N-yl)propionamide,
N-(2-dimethylaminoethyl)-3-(1,5-didesoxy-1,5-imino-D-mannit-N-yl)propionamide,
N-[2-(3-methoxyphenyl)ethyl]-3-(1,5-didesoxy-1,5-imino-D-mannit-N-yl)propionamide,
N-(2-pyridylmethyl)-3-(1,5-didesoxy-1,5-imino-D-mannit-N-yl)propionamide,
3-(1,5-didesoxy-1,5-imino-D-mannit-N-yl)-propionyl-(4-cinnamyl)piperazine, and
3-(1,5-didesoxy-1,5-imino-D-mannit-N-yl)-4-(2-hydroxyethoxy) anilide.

5. Medicaments according to Claims 1 to 4 for combating viral diseases.

6. Medicaments according to Claims 1 to 4 for combating retroviral diseases.

7. Medicaments according to Claims 1 to 4 for combating HIV infections.

**Revendications**

1. Médicament contenant en combinaison de l'interféron et des composés de la formule générale (I)

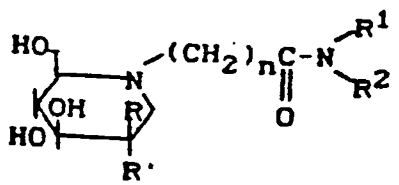

(I)

dans laquelle le radical

R ou R′ représente un groupe hydroxyle, tandis que l'autre, soit R′, soit R, représente de l'hydrogène,

n — représente un nombre de 2 à 6

$R^1$ — représente de l'hydrogène, ou
— un radical alkyle à chaîne linéaire ou ramifiée avec 3 atomes de carbone au maximum, ou bien représente un radical benzyle,
et

$R^2$ — représente un radical alkyle à chaîne linéaire ou ramifiée ou avec 8 atomes de carbone au maximum, cet alkyle pouvant être substitué par un radical aryle à 6 à 10 atomes de carbone, dans lequel le radical aryle peut porter jusqu'à 4 substituants différents ou identiques, compris dans la série fluoro, chloro, bromo, iodo, cyano, nitro, alkyle, alcoxy ou alkylthio comportant chacun jusqu'à 6 atomes de carbone, dioxyméthylène, dioxyéthylène, trifluorométhyle, trifluorométhoxy, difluorométhoxy, carboxy ou alcoxycarbonyle avec 4 atomes de carbone au maximum ou qui peut être substitué par un radical pyridyle, thiényle, furyle, pyrimidyle, pyridazinyle, pyrazinyle ou quinolyle ou
— représente un radical cycloalkyle avec 3 à 7 atomes de carbone, ou
— représente un radical aryle à 6 à 10 atomes de carbone dans lequel le radical aryle peut être substitué jusqu'à 4 fois de manière identique ou différente par un fluor, un chlore, un brome, un iode, un cyano, un alkyle à 6 atomes de carbone au maximum, un alkyloxy à 6 atomes de carbone au maximum, un alkylsulfonyle avec 4 atomes de carbone au maximum, un trifluoro-méthyle, un trifluorométhoxy, un difluoro-méthylène, un difluorométhoxy, un trifluoro-méthylthio, un dioxyméthylène, un dioxyéthylène, par un cycloalkyle à 3 à 7 atomes de carbone, par un radical carboxy ou alcoxycarbonyle à 4 atomes de carbone au maximum, par un radical aryloxy à 6 à 10 atomes de carbone, dans lequel le radical aryle peut porter jusqu'à 4 substituants identiques ou différents compris dans la série fluoro, chloro, bromo, iodo, cyano, hydroxy, nitro, alkyle, alcoxy, alkylthio avec chaque fois jusqu'à 6 atomes de carbone, un dioxyméthylène, dioxyéthylène, trifluorométhyle, trifluorométhoxy ou difluorométhoxy, ou par un radical alcoxy halogéné avec 6 atomes de carbone au maximum et au maximum 5 atomes de fluor et/ou 3 atomes de chlore, ou par un radical hydroxyalcoxy, hydroxyalkyle ou cyanoalkyle avec chaque fois 8 atomes de carbone au maximum ou bien par un radical alkényloxy avec 6 atomes de carbone au maximum ou par un groupe —$XR^3$, dans lequel

X — représente une chaîne alcénylène ou alkylène linéaire ou ramifiée, avec au maximum 8 atomes de

carbone,
et

$R^3$ représente un radical aryle avec 6 à 10 atomes de carbone, dans lequel le radical aryle peut porter jusqu'à 4 substituants identiques ou différents de la série fluoro chloro, bromo, iodo, cyano, alkyle, alcoxy, alkylthio avec chaque fois jusqu'à 6 atomes de carbone, nitro, dioxyméthylène, dioxyéthylène, trifluorométhyle,trifluorométhoxy, difluorométhoxy ou trifluorométhylthio ou
— représente un cycle hétérocyclique saturé ou insaturé à 5 ou 6 éléments, qui peut être benzocondensé et qui peut contenir comme hétéroatomes de l'oxygène, du soufre ou 2 atomes d'azote au maximum, ou
— représente un radical hydroxy, carboxy, alcoxycarbonyle à 6 atomes de carbone au maximum, alkylcarbonyloxy à 18 atomes de carbone au maximum, cycloalkyle à 3 à 6 atomes de carbone, ou bien
— représente un groupe —$OSO_3$ H, —CONH-$NH_2$, —$CONH_2$ ou amino, alkylamino ou dialkylamino avec chaque fois 6 atomes de carbone au maximum par groupe alkyle ou

représente un radical alkyle ou hydroxy-alkyle avec 8 atomes de carbone au maximum,
ou bien

$R^1$ et $R^2$ forment avec l'atome d'azote un cycle à 5 à 6 éléments qui peut être interrompu par de l'oxygène ou le groupe —$NR^4$ et qui peut être constitué par un radical alkyle à 4 atomes de carbone au maximum, un radical hydroxy, phényle, carboxy ou alcoxycarbonyle à 4 atomes de carbone au maximum,
dans lequel

$R^4$ — représente un radical aryle à 6 à 10 atomes de carbone, dans lequel le radical aryle peut porter 4 substituants identiques ou différents faisant partie du groupe fluoro, chloro, iodo, cyano, nitro, hydroxy, alkyle, alcoxy, ~~alkylthio~~ ayant chacun 6 atomes de carbone au maximum, dioxyméthylène, dioxyéthylène, trifluorométhyle, trifluorométhoxy ou difluorométhoxy ou bien
— alcoxycarbonyle à 6 atomes de carbone au maximum ou
— représente un radical pyridyle, pyrimidyle, furyle, thiényle, pyrazinyle, pyridazinyle ou quinolyle, ou représente un radical cycloalkyle à 3 à 7 atomes de carbone ou
— représente un radical alkyle ou alkényle à 6 atomes de carbone au maximum, qui peut être substitué deux fois au maximum et de manière identique ou différente par un radical phényle, éventuellement substitué par le chlore, le dioxyméthylène ou le trifluorométhyle, un radical hydroxy, amino, alkylamino, dialkylamino, avec chaque fois jusqu'à 3 atomes de carbone par groupe alkyle, un radical fluoro, chloro, bromo, cycloalkyle à 3 à 6 atomes de carbone, par un radical carboxy ou alcoxycarbonyle à 6 atomes de carbone au maximum, par un radical pyridyle, pyrimidyle, pyrazinyle, morpholinyle, thiomorpholinyle ou pyrrolidinyle, par un radical morpholinocarbonyle, un radical alkylaminocarbonyle, dialkylaminocarbonyle ou phénylalkylaminocarbonyle avec chaque fois 6 atomes de carbone au maximum par groupe alkyle.

2. Médicament contenant en combinaison de l'interféron et des composés de la formule générale (I) selon la revendication 1, dans laquelle

n — représente un nombre de 2 à 4,
$R^1$ — représente de l'hydrogène
et

$R^2$ — représente un radical alkyle à chaîne linéaire ou ramifiée à 6 atomes de carbone au maximum, qui peut être substitué par un radical pyridyle ou par un phényle, dans lequel le radical phényle peut porter jusqu'à 3 substituants identiques ou différents faisant partie de la série fluoro,chloro, bromo, hydroxy, alkyle, alcoxy à 4 atomes de carbone au maximum, dioxyméthylène, dioxyéthylène ou carboxy, ou
— représente un radical cyclopentyle, cyclohexyle ou cycloheptyle, ou
— représente un radical phényle qui peut être substitué 3 fois de manière identique ou différente par du fluor, du chlore, du brome, un cyano, un alkyle à 4 atomes de carbone au maximum, un hydroxyalkyle ou un hydroxyalcoxy à 4 atomes de carbone au maximum, un trifluorométhyle, un trifluorométhoxy,un dioxyméthylène, un dioxyéthylène, par un radical phénoxy éventuellement substitué dans l'aromatique par du fluor, du chlore, du brome, un hydroxy, un alkyle, un alcoxy à 4 atomes de carbone au maximum,

un dioxyméthylène, un trifluorométhyle ou un carboxy, ou par un alkényloxy à chaîne linéaire ou ramifiée avec 4 atomes de carbone au maximum ou par un groupe —$XR^3$ dans lequel

X — représente une chaîne alkylène linéaire ou ramifiée à 6 atomes de carbone au maximum et

$R^3$ — représente un phényle substitué 3 fois au maximum de manière identique ou différente par du fluor, du chlore, du brome, un hydroxy, un alkyle, un alcoxy à 4 atomes de carbone au maximum, un dioxyméthylène, un trifluorométhyle ou un carboxy, ou
— représente un radical pyridyle, morpholinyle ou pipéridinyle, ou
— représente un radical hydroxy, carboxy, alcoxycarbonyle à 4 atomes de carbone au maximum ou un radical cyclopropyle, un radical cyclopentyle ou un radical cyclohexyle, ou
— représente un radical amino, alkylamino ou dialkylamino avec chaque fois 4 atomes de carbone au maximum par groupe alkyle,
ou bien

$R^1$ et $R^2$ forment, avec l'azote, un cycle des formules

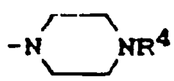 , 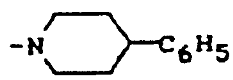 , 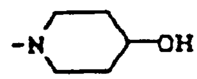 ,

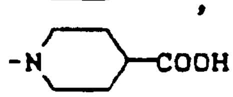 . ou 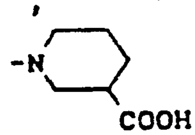

dans lesquelles

$R^4$ — représente un radical phényle substitué éventuellement 3 fois au maximum et de manière identique ou différente par du fluor, du chlore, du brome, un hydroxy, un alkyle, un alcoxy à 4 atomes de carbone au maximum, dioxyméthylène, trifluorométhyle ou un carboxy, ou
— représente un radical alcoxycarbonyle à 4 atomes de carbone au maximum, ou
— représente un radical pyridyle ou pyrimidyle, ou représente
— un radical cyclopropyle, cyclopentyle ou cyclohexyle, ou
— représente un radical alkyle ou alkényle à chaîne linéaire ou ramifiée à 4 atomes de carbone au maximum qui peut être substitué 2 fois au maximum de manière identique ou différente par un radical phényle substitué éventuellement par du chlore, un trifluorométhyle ou un dioxyméthylène, par un radical hydroxy, amino, méthylamino, diméthylamino, fluoro, chloro, cyclopropyle, cyclopentyle, cyclohexyle ou alcoxycarbonyle à 4 atomes de carbone au maximum, par un radical morpholinyle, pyrrolidinyle, morpholino-carbonyle, dialkylamino ou phénylalkylamino avec chacun 4 atomes de carbone au maximum.

3. Médicament contenant en combinaison de l'interféron et des composés de la formule générale (I) selon la revendication 1, dans laquelle

n — représente le nombre 2,
$R^1$ — représente de l'hydrogène,
et

$R^2$ — représente un radical phényle qui peut être substitué 3 fois au maximum de manière identique ou différente par du fluor, du chlore, du brome, un cyano, un alkyle à 4 atomes de carbone au maximum, un hydroxyalkyle, un hydroxyalcoxy à 4 atomes de carbone au maximum, un alkylthio à 4 atomes de carbone au maximum, un trifluorométhyle, un trifluorométhoxy,
$R^1$ et $R^2$ forment avec l'azote un cycle de la formule

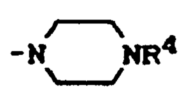

dans laquelle

$R^4$ représente un radical phényle substitué éventuellement deux fois au maximum de manière identique ou différente par du fluor, du chlore, du brome, un hydroxy, un alkyle, un alcoxy à 4 atomes de carbone au maximum, ou
— un radical alcoxycarbonyle à 4 atomes de carbone au maximum, ou
— un radical cyclopropyle, cyclopentyle, cyclohexyle, ou
— représente un radical alkyle ou alkényle à chaîne linéaire ou ramifiée à 4 atomes de carbone au maximum qui peut être substitué 2 fois au maximum de manière identique ou différente par un radical phényle substitué éventuellement par du chlore, un trifluorométhyle ou un dioxyméthylène, par un radical hydroxy, un amino, diméthylamino, cyclopropyle ou alcoxycarbonyle à 4 atomes de carbone au maximum, par un radical morpholinyle ou pyrrolidinyle.

4. Médicament contenant en combinaison de l'interféron et des composés du groupe suivant :
(4-méthoxy)-anilide de l'acide
3-(1,5-didésoxy-1,5--imino-D-glucite-N-yl)-propionique, (4-méthoxycarbonyl)-anilide de l'acide
3,(1,5-didésoxy-1,5-imino-D-glucite-N-yl)-propionique, 4-(4-pyridylméthoxy)anilide de l'acide
3-(1,5-didésoxy-1,5-imino-D-glucite-N-yl)-propionique, (4-benzyloxy)-anilide de l'acide
3-(1,5-didésoxy-1,5-imino-D-glucite-N-yl)-propionique, (4-trifluorométhoxy)-anilide de l'acide
3-(1,5-didésoxy-1,5-imino-D-glucite-N-yl)-propionique, 4-(2-oxocyclohexyloxy)-anilide de l'acide
3-(1,5-didésoxy-1,5-imino-D-glucite-N-yl)-propionique, 4-tert.butyl-anilide de l'acide
3-(1,5-didésoxy-1,5-imino-D-glucite-N-yl)-propionique, 4-(6-méthyl-heptyloxy)-anilide de l'acide
3-(1,5-didésoxy-1,5-imino-D-glucite-N-yl)-propionique, 4-(2-N,N-diéthyl-amino-éthoxy)-anilide de l'acide
3-(1,5-didésoxy-1,5-imino-D-glucite-N-yl)-propionique, 4-allyloxy-anilide de l'acide
3-(1,5-didésoxy-1,5-imino-D-glucite-N-yl)-propionique, 4-trifluorométhyl-anilide de l'acide
3-(1,5-didésoxy-1,5-imino-D-glucite-N-yl)-propionique, 4-(2-chloro-1,1,2-trifluoroéthoxy)-anilide de l'acide
3-(1,5-didésoxy-1,5-imino-D-glucite-N-yl)-propionique, (4-isopropyl)-anilide de l'acide
3-(1,5-didésoxy-1,5-imino-D-glucite-N-yl)-propionique, 2-isopropyl-anilide de l'acide
3-(1,5-didésoxy-1,5-imino-D-glucite-N-yl)-propionique, 4-fluoro-anilide de l'acide
3-(1,5-didésoxy-1,5-imino-D-glucite-N-yl)-propionique, 2-fluoro-anilide de l'acide
3-(1,5-didésoxy-1,5-imino-D-glucite-N-yl)-propionique,
1-[3-(1,5-didésoxy-1,5-imino-D-glucite-N-yl)-propionyl] 2,3-dihydro-indole,
2,4-difluoroanilide de l'acide
3-(1,5-didésoxy-1,5-imino-D-glucite-N-yl)-propionique, 2-trifluorométhyl-anilide de l'acide
3-(1,5-didésoxy-1,5-imino-D-glucite-N-yl)-propionique, 2,6-difluoroanilide de l'acide
3-(1,5-didésoxy-1,5-imino-D-glucite-N-yl)-propionique, (adamantane-1-yl)-amide de l'acide
3-(1,5-didésoxy-1,5-imino-D-glucite-N-yl)-propionique, 3,4,5-triméthoxy-anilide de l'acide
3-(1,5-didésoxy-1,5-imino-D-glucite-N-yl)-propionique, 3,4-diméthoxy-anilide de l'acide
3-(1,5-didésoxy-1,5-imino-D-glucite-N-yl)-propionique, 3,5-diméthoxy-anilide de l'acide
3-(1,5-didésoxy-1,5-imino-D-glucite-N-yl)-propionique, 2,4,6 tribromoanilide de l'acide
3-(1,5-didésoxy-1,5-imino-D-glucite-N-yl)-propionique, 3-cyano-anilide de l'acide
3-(1,5-didésoxy-1,5-imino-D-glucite-N-yl)-propionique, 4-cyclohexyl-anilide de l'acide
3-(1,5-didésoxy-1,5-imino-D-glucite-N-yl)-propionique, 3,4-éthylènedioxy-anilide de l'acide
3-(1,5-didésoxy-1,5-imino-D-glucite-N-yl)-propionique, 2-bromoanilide de l'acide
3-(1,5-didésoxy-1,5-imino-D-glucite-N-yl)-propionique, (4-trifluorométhoxy)anilide de l'acide
3-(1,5-didésoxy-1,5-imino-D-glucite-N-yl)-propionique, 3,4-méthylènedioxy-anilide de l'acide
3-(1,5-didésoxy-1,5-imino-D-glucite-N-yl)-propionique, 2-méthylthio-anilide de l'acide
3-(1,5-didésoxy-1,5-imino-D-glucite-N-yl)-propionique, 3-(1,5-didésoxy-1,5-imino-D-glucite-N-yl)-propionyl-4-(4-trifluorométhyl-phényl)-pipérazine, anilide de l'acide
3-(1,5-didésoxy-1,5-imino-D-glucite-N-yl)-propionique, 4-éthoxy-anilide de l'acide
3-(1,5-didésoxy-1,5-imino-D-glucite-N-yl)-propionique, 2-diméthylaminoéthyl-amide de l'acide
3-(1,5-didésoxy-1,5-imino-D-glucite-N-yl)-propionique, [2-(3-méthoxyphényl)éthyl]-amide de l'acide
3-(1,5-didésoxy-1,5-imino-D-glucite-N-yl)-propionique, 2-pyridilméthyl-amide de l'acide
3-(1,5-didésoxy-1,5-imino-D-glucite-N-yl)-propionique, 3-(1,5-didésoxy-1,5-imino-D-glucite-N-yl)-propionyl(4-cinnamyl)-pipérazine,
4-(2-hydroxyéthoxy)-anilide de l'acide
3-(1,5-didésoxy-1,5-imino-D-glucite-N-yl)-propionique, (4-méthoxy)-anilide de l'acide
3-(1,5-didésoxy-1,5-imino-D-mannite-N-yl)-propionique, (4-méthoxycarbonyl)-anilide de l'acide

3-(1,5-didésoxy-1,5-imino-D-mannite-N-yl)-propionique, 4-(4-pyridylméthoxy)-anilide de l'acide
3-(1,5-didésoxy-1,5-imino-D-mannite-N-yl)-propionique, 4-benzyloxy-anilide de l'acide
3-(1,5-didésoxy-1,5-imino-D-mannite-N-yl)-propionique, 4-trifluoromethoxy-anilide de l'acide
3-(1,5-didesoxy-1,5-imino-D-mannite-N-yl)-propionique, 4-(2-oxocyclohexyloxy)-anilide de l'acide
3-(1,5-didésoxy-1,5-imino-D-mannite-N-yl)-propionique, 4-tert.butyl-anilide de l'acide
3-(1,5-didésoxy-1,5-imino-D-mannite-N-yl)-propionique, 4-(6-méthyl-heptyloxy)-anilide de l'acide
3-(1,5-didésoxy-1,5-imino-D-mannite-N-yl)-propionique, 4-(2-N,N-diéthyl-amino-éthoxy)-anilide de l'acide
3-(1,5-didésoxy-1,5-imino-D-mannite-N-yl)-propionique, 4-allyloxy-anilide de l'acide
3-(1,5-didésoxy-1,5-imino-D-mannite-N-yl)-propionique, 4-trifluorométhyl-anilide de l'acide
3-(1,5-didésoxy-1,5-imino-D-mannite-N-yl)-propionique, 4-(2-chloro-1,1,2-trifluoroéthoxy)-anilide de l'acide
3-(1,5-didésoxy-1,5-imino-D-mannite-N-yl)-propionique, 4-isopropyl-anilide de l'acide
3-(1,5-didésoxy-1,5-imino-D-mannite-N-yl)-propionique, 2-isopropyl-anilide de l'acide
3-(1,5-didésoxy-1,5-imino-D-mannite-N-yl)-propionique, 4-fluoro-anilide de l'acide
3-(1,5-didésoxy-1,5-imino-D-mannite-N-yl)propionique, 2-fluoro-anilide de l'acide
3-(1,5-didésoxy-1,5-imino-D-mannite-N-yl)-propionique, 1-[3-(1,5-didésoxy-1,5-imino-D-mannite-N-yl)-propionyl]-2,3-dihydro-indole,
2,4-difluoroanilide de l'acide
3-(1,5-didésoxy-1,5-imino-D-mannite-N-yl)-propionique, 2-trifluorométhyl-anilide de l'acide
3-(1,5-didésoxy-1,5-imino-D-mannite-N-yl)-propionique, 2,6-difluoroanilide de l'acide
3-(1,5-didésoxy-1,5-imino-D-mannite-N-yl)-propionique, (adamantane-1-yl)-amide de l'acide
3-(1,5-didésoxy-1,5-imino-D-mannite-N-yl)-propionique, 3,4,5-triméthoxy-anilide de l'acide
3-(1,5-didésoxy-1,5-imino-D-mannite-N-yl)-propionique, 3,4-diméthoxy-anilide de l'acide
3-(1,5-didésoxy-1,5-imino-D-mannite-N-yl)-propionique, 3,5-diméthoxy-anilide de l'acide
3-(1,5-didésoxy-1,5-imino-D-mannite-N-yl)propionique, 2,4,6-tribromoanilide de l'acide
3-(1,5-didésoxy-1,5-imino-D-mannite-N-yl)-propionique, 3-cyano-anilide de l'acide
3-(1,5-didésoxy-1,5-imino-D-mannite-N-yl)-propionique,4-cyclohexyl-anilide de l'acide
3-(1,5-didésoxy-1,5-imino-D-mannite-N-yl)-propionique, 3,4-éthylènedioxy-anilide de l'acide
3-(1,5-didésoxy-1,5-imino-D-mannite-N-yl)-propionique, 2-bromo-anilide de l'acide
3-(1,5-didésoxy-1,5-imino-D-mannite-N-yl)-propionique, 3,4-méthylènedioxy-anilide de l'acide
3-(1,5-didésoxy-1,5-imino-D-mannite-N-yl)-propionique, 2-méthylthio-anilide de l'acide
3-(1,5-didésoxy-1,5-imino-D-mannite-N-yl)-propionique, 3-(1,5-didésoxy-1,5-imino-D-mannite-N-yl)-propionyl-4-(4-trifluorométhyl-phényl)-pipérazine,
Anilide de l'acide
3-(1,5-didésoxy-1,5-imino-D-mannite-N-yl)propionique, 4-éthoxy-anilide de l'acide
3-(1,5-didésoxy-1,5-imino-D-mannite-N-yl)-propionique, 2-diméthylaminoéthyl-amide de l'acide
3-(1,5-didésoxy-1,5-imino-D-mannite-N-yl)-propionique, (2-(3-méthoxyphényl)éthyl)-amide de l'acide
3-(1,5-didésoxy-1,5-imino-D-mannite-N-yl)-propionique, 2-pyridylméthyl-amide de l'acide
3-(1,5-didésoxy-1,5-imino-D-mannite-N-yl)-propionique, 3-(1,5-didésoxy-1,5-imino-D-mannite-N-yl)-propionyl-(4-cinnamyl)-pipérazine, et
3-(1,5-didésoxy-1,5-imino-D-mannite-N-yl)-4-(2-hydroxyéthoxy)anilide.

5. Médicament selon les revendications 1 à 4, pour le traitement des affections virales.

6. Médicament selon les revendications 1 à 4, pour le traitement des affections rétrovirales.

7. Médicament selon les revendications 1 à 4, pour le traitement des affections HIV.